# EUROPEAN PATENT SPECIFICATION

(11) **EP 0 925 798 B1**
(45) Date of publication and mention of the grant of the patent: **14.05.2003**
(21) Application number: 98124606.9
(22) Date of filing: 23.12.1998
(51) Int. Cl.: A61M 5/315

(54) **Easy-slip plunger/plunger rod assembly for a syringe or a cartridge**
Kolben- und Stangenzusammensetzung für Spritze oder Kartusche mit guter Gleitfähigkeit
Montage de tige et piston pour seringue ou cartouche avec glissement léger

(30) Priority: 24.12.1997 US 998204
(43) Date of publication of application: 30.06.1999
(73) Proprietor: Bracco International B.V., 1077 ZX Amsterdam (NL)
(72) Inventor: Gabbard, Mark E., Salisbury, MD 21801 (US); Niedospital, John J. Jr., Burlington, NJ 08016 (US); Gabbard, Timothy J., Salisbury, MD 21804 (US)
(74) Representative: Gervasi, Gemma, Dr.

(56) References cited:
- EP-A- 0 328 504
- WO-A-93/04951
- BE-A- 715 635
- GB-A- 743 789
- US-A- 4 030 496
- US-A- 4 074 715
- US-A- 4 201 209
- US-A- 5 226 897
- US-A- 5 453 093

## Description

### Field of the Invention

This invention relates to plunger rod and plunger combinations utilized in syringes and cartridges for the introduction or withdrawal of fluids to and from a patient. More particularly, the invention relates to glass or plastic syringes and cartridges which may be pre-filled with a pharmaceutical fluid for injections to a patient.

### Background of the invention

Syringes and cartridges made of glass or polymeric material for delivery of fluids to and from a patient have been proposed and utilized by the prior art, and have achieved a highly developed state. Various requirements related to specific delivery systems have also been addressed. While specific requirements of fluid delivery to and from a patient may vary, means of delivery remain essentially the same and may be characterized by the following general description of a syringe.

A syringe comprises:
a) a cylindrical barrel having a proximal end designed for receiving a plunger with or without a plunger rod removably attached to the plunger or being integral with the plunger, and a distal end adapted to mount a needle or luer connector thereon; and
b) a plunger slideably mounted in the barrel.

The plunger is inserted into the barrel at the proximal end of the syringe and thus when fluid is contained in the barrel it may be expelled by pushing the plunger in the barrel towards its distal end; or when the syringe is used to withdraw fluid from a patient, the plunger located at the distal end of the barrel is pulled towards the proximal end of the syringe thereby drawing fluid into the barrel. Since a fluid-tight seal is necessary between the plunger and the inside wall of the barrel, a resilient rubber tip is positioned on the distal end of the plunger, or typically, the plunger is made of resilient rubber-like material. In some of the syringes of the prior art the rubber tip has been replaced with a generally flat, circular disk as part of the plunger.

In order to assure air-tight seal between the inside wall of the syringe barrel and the plunger, prior art plungers are manufactured with a larger outside diameter than the inside diameter of the syringe barrels. When the plunger is introduced into the syringe barrel, it is sufficiently compressed to provide adequate pressure between the inside wall of the syringe and the plunger to seal the interface and withstand the challenges of filling, injecting and withdrawing fluids using the syringe without leakage.

In addition to a leakage-proof seal, another requirement in the syringe/plunger combination is the chemical stability of both the syringe and the plunger. While syringes being made of glass or thermoplastic materials are sufficiently chemically inert to pharmaceutical and biological fluids contained therein, the plungers made of natural rubber or butyl rubber have some undesirable properties. The rubber contains additional chemical components such as fillers and accelerators introduced during the curing process which tend to exude to the surface of the plunger during the contact between the plunger and the fluid contained in the syringe. Such exudate is undesirable in an injection or when a biological fluid, such as blood, is withdrawn from a patient for testing purposes. The problem is further aggravated when there is a long-term storage of the content of the pharmaceutical /biological fluid in the syringe. Recognizing the problem of contamination caused by exudates from plungers made of rubber, the prior art has provided plungers made of thermoplastic materials which do not contain the additives that rubber plungers contain. However, thermoplastic materials are not as resilient as rubbers and the seals formed between thermoplastic plungers and the inside walls of syringes tend to be inadequate in some circumstances. Also, over a period of time on storage the thermoplastic plunger may achieve a compression stage wherein the outside diameter of the plunger is reduced thereby no longer capable of forming a tight seal between it and the inside wall of the syringe.

In addition to the tendency of leakage, the thermoplastic plunger does not slide smoothly in the syringe barrel and requires the exertion of excessive force on the plunger rod to move the plunger in the barrel. The exertion of excessive force on the plunger rod may result in uneven delivery of the fluid to the patient or insertion of the needle into a vein or tissue area to an undesirable depth.

In both the rubber and thermoplastic plungers a relatively large compressive force must be exerted on the plungers by the syringe barrel to provide for a tight, leakproof seal. This quality of the seal, however, makes the movement of the plunger difficult. To remedy the problem the prior art used lubricants to reduce friction and drag between the plunger and the inside surface of the syringe barrel. The use of such lubricants, however, is also undesirable with certain parenteral fluids which tend to disperse or dissolve in the parenteral fluids thereby contaminating the parenteral fluids. Attempts to avoid the use of lubricants included the use of various plunger configurations, such as plungers that were provided with one or more ribs projecting forwardly or rearwardly in the barrel to reduce the frictional drag between the plunger and the inside surface of the barrel.

As specific documents of the state of the art, some examples are the following:
■ US patent No. 4,543,093 relates to a variable sealing pressure plunger rod assembly comprising a plunger rod and a flexible thermoplastic stopper; the thermoplastic stopper including an inside cavity to receive the distal end portion of the plunger rod;
■ US patent No. 4,673,396 relates to a syringe cartridge used in conjunction with blood gas measurements; the syringe cartridge contains a plunger piston 14 having a dome 62; a pair of seal rings 65 on the exterior of the plunger piston are provided to engage the interior wall of the cartridge;
■ US patent No. 5,314,416 discloses a low friction syringe assembly comprising: a plunger rod and a plunger tip which are designed to provide a low friction resistance against the interior surface of the syringe barrel while the syringe barrel is at rest but which will increase the sealing pressure between the plunger tip and the interior surface of the syringe barrel in proportion to the amount of force applied to the plunger rod;
■ US patent No. 5,413,563 pertains to a pre-filled syringe having a plunger, plunger insert and plunger rod; the plunger insert 68 exerts force against bottom rim 39 of plunger 30 during manual aspiration;
■ US patent No. 5,624,405 relates to a pre-filled syringe and syringe tip assembly comprising: a plunger 6, plunger rod 62 and a gasket 61 fitted on the top of the plunger rod 62 so as to move smoothly along the lumen of the syringe body 1.

US 5 453 093 and WO9304951 disclose a dental syringe and a non-reactive composite sealing barrier respectively and disclose the preamble of independant claims 1 and 22 respectively.
All of the above-cited references use a plunger rod and a plunger assembly in the barrel of a syringe cartridge or vial and all are, at least in part, directed to a leak-proof seal and easy sliding objective. While fluid tightness and sliding property have improved with these attempts, it appears that improvement in one of these properties is not quite achieved without corresponding decrease in the other property: increasing fluid tightness tends to result in decreasing sliding property, while increasing sliding property tends to result in decreasing fluid tightness. None of the references cited disclose or suggest combination of plunger rod and plunger having the desirable characteristics disclosed in the present invention.

The present invention addresses the following main requirements in a syringe or cartridge:
reduction of breakaway forces;
reduction of running forces;
insuring against leakage; and
reduction of contact area between the medical fluid and the elastomeric rubber plunger.

These and other related requirements will be addressed in the embodiments provided by the present invention the description of which follows.

### SUMMARY OF THE INVENTION

The present invention provides a syringe or a prefilled cartridge for medical use designed for injecting a fluid into a site or, in case of the syringe, also for withdrawing a fluid from a site comprising a syringe or cartridge barrel and a plunger, slideably mounted in said barrel, comprising a rigid plunger rod tip/elastomeric plunger ring assembly.
A particular aspect of the invention is a syringe for medical use designed for injecting a fluid into a site or withdrawing a fluid from a site as disclosed in independant claim 1.
Another particular aspect of the invention is a prefilled cartridge for medical use designed for injecting a fluid into a site as disclosed in independant claim 22.

The non-slideable seal formed between the cylindrical shaft having the groove or recess therein and the elastomeric plunger ring insures against leakage between the two dissimilar materials.

The rigid plunger rod tip, preferably made of thermoplastic material does not provide a seal: it freely moves within the barrel of the syringe. However it serves the important function of interfacing with the content of the syringe or cartridge barrel. Since its material of construction is inert to medical fluids there is no contamination of the medical fluids thereby. The plunger rod tip may provide an interface of from at least 95% to about 99% with the medical fluid.

The elastomeric plunger ring having plunger ring ribs thereon forms the secondary slideable seal between the plunger rind and the inside of the syringe or cartridge barrel. The contact or interface between the plunger ring ribs and the medical fluid is minimal, thereby contamination from the elastomeric plunger ring ribs by leaching is greatly reduced.

### BRIEF DESCRIPTION OF THE DRAWINGS

- FIG. 1: is a perspective view of the assembled syringe containing a plunger ring, plunger rod and plunger rod tip according to the present invention;
- FIG. 2: is a longitudinal cross-section of the plunger rod removed from the syringe taken along the line 2-2 of FIG. 1;
- FIG. 3A: is a perspective view of the plunger ring shown in FIG. 1;
- FIG. 3B: is a side elevational view of the plunger ring shown in FIG. 3A;
- FIG. 4A: shows a longitudinal cross-section of the plunger rod tip with plunger ring thereon, wherein the plunger rod tip has internal female threads for attachment to a male-threaded plunger rod;
- FIG. 4B: shows a top plan view of the plunger rod tip with plunger ring thereon shown in FIG. 4A;
- FIG. 5A: shows the longitudinal cross-section of another plunger rod tip designed to carry one internal seal plunger ring thereon wherein the plunger rod tip has internal female threads for attachment to a male-threaded plunger rod;
- FIG. 5B: shows a top plan view of the plunger rod tip shown in FIG. 5A;
- FIG. 6A: shows the longitudinal cross-section of still another plunger rod tip, with accentuated contours, designed to carry one internal seal plunger ring thereon, wherein the plunger rod tip has internal female threads for attachment to a male-threaded plunger rod;
- FIG. 6B: shows a top plan view of the plunger rod tip shown in FIG. 6A;
- FIG. 7A: shows the longitudinal cross-section of still another plunger rod tip designed to carry two internal seals plunger ring thereon, wherein the plunger rod tip has internal female threads for attachment to a male-threaded plunger rod;
- FIG. 7B: shows a top plan view of the plunger rod tip shown in FIG. 7A;
- FIG. 8: shows the longitudinal cross-section of a plunger ring having one internal seal;
- FIG. 9: shows the longitudinal cross-section of the plunger ring having one internal seal shown in FIG. 8 and the direction of various pressures exerted on the plunger ring in use;
- FIG. 10A: shows the longitudinal cross-section of still another plunger ring having one internal seal in place on the plunger rod tip;
- FIG. 10B: shows a side elevational view of the cross-section of the plunger ring in place on the plunger rod tip shown in FIG. 10A;
- FIG. 11A: shows the longitudinal cross-section of still another plunger ring having one internal seal in place on the plunger rod tip and is designed to provide leak-proof protection in one direction;
- FIG. 11B: shows a side elevational view of the cross-section of the plunger ring in place on the plunger rod tip shown in FIG. 11A;
- FIG. 12A: shows the longitudinal cross-section of still another plunger ring having one internal seal and is designed to provide leak-proof protection in both directions;
- FIG. 12B: shows a side elevational view of the cross-section of the plunger ring in place on the plunger rod tip shown in FIG. 12A;
- FIG. 13A: shows the longitudinal cross-section of still another plunger ring in place on the plunger rod tip having one internal seal and is designed to provide leak-proof protection in both directions;
- FIG. 13B: shows a side elevational view of the cross-section of the plunger ring in place on the plunger rod tip shown in FIG. 13A;
- FIG. 14: is a longitudinal fragmentary cross-section of a typical prior art syringe, plunger and plunger rod;
- FIG. 15: is an enlarged fragmentary cross-section of the prior art plunger and plunge rod of FIG. 14;
- FIG. 16: shows a graph of breakaway forces between the inside wall of the barrel and a butyl rubber plunger with silicone of the prior art;
- FIG. 17: shows a graph of breakaway forces between the inside wall of the barrel and the butyl rubber plunger without silicone of the prior art;
- FIG. 18: shows a graph of running forces between the inside wall of the barrel and the butyl rubber plunger without silicone of the prior art;
- FIG. 19: shows a graph of running forces between the inside wall of the barrel and the butyl rubber plunger with silicone of the prior art;
- FIG. 20: shows a graph of average breakaway forces between the inside wall of the barrel and butyl rubber plunger with silicone of the prior art and the average breakaway forces between the inside wall of the barrel and the plunger rod tip/plunger ring combination with silicone of the present invention;
- FIG. 21: shows a graph of average breakaway forces between the inside wall of the barrel and butyl rubber plunger without silicone of the prior art and the average breakaway forces between the inside wall of the barrel and the plunger rod tip/plunger ring combination without silicone, wherein the plunger ring is made of Silicone(50), Nitrial(65), Nitrial(45), Viton(65) or Butyl(52) rubbers;
- FIG. 22: shows a graph of running forces between the inside wall of the barrel and the plunger rod tip/plunger ring combination with silicone of the present invention;
- FIG. 23: shows a graph of running forces between the inside wall of the barrel and the plunger of the prior art made of butyl rubber with silicone, versus a graph of running forces between the inside wall of the barrel and the plunger rod tip/plunger ring combination made of butyl rubber with silicone of the present invention;
- FIG. 24: shows a graph of running forces between the inside wall of the barrel and the plunger made of butyl rubber without silicone of the prior art;
- FIG. 25: shows a graph of running forces between the inside wall of the barrel and the plunger rod tip/plunger ring combination without silicone of the present invention; and
- FIG. 26: shows a graph of running forces between the inside wall of a barrel and the plunger of the prior art made of butyl rubber without silicone, versus a graph of running forces between the inside wall of the barrel and the plunger rod tip/plunger ring combination made of butyl rubber without silicone.

### DETAILED DESCRIPTION OF THE INVENTION

The present invention is directed to syringes and cartridges used to deliver fluids into a site, such as liquid pharmaceutical compositions and diagnostic contrast media delivered into a mammalian patient subcutaneously, intramuscularly or intravenously depending upon the particular medication to be administered. The barrel of the syringe is made of glass or polymeric materials and is equipped with a needle or luer connector having a tubing conduit attached thereto at the distal, tapered end thereof through which the fluids are delivered into the desired site. The syringes or cartridges may be pre-filled and sterilized ready for use, or they may be filled from a container, such as a vial, just prior to use. They may be used manually or in connection with power injectors well-known in the art.

The syringes or cartridges of the present invention may also be used to withdraw biological fluids from a patient, such as blood or tissue, for testing or other medical purposes.

The design of the thermoplastic plunger rod tip and the elastomeric plunger ring assembly used in the syringe or cartridge results in the following desirable characteristics:
reduction of breakaway forces;
reduction of running forces;
insuring against leakage; and
reduction of contact area between the medical fluid and the rubber plunger.

The invention will now be described in reference to syringes, however, it is to be noted that the invention encompasses cartridges as well. Reference is now made to the drawings in describing the syringes in general and the thermoplastic plunger rod tip/elastomeric plunger ring assembly in particular detail which together constitute the invention.

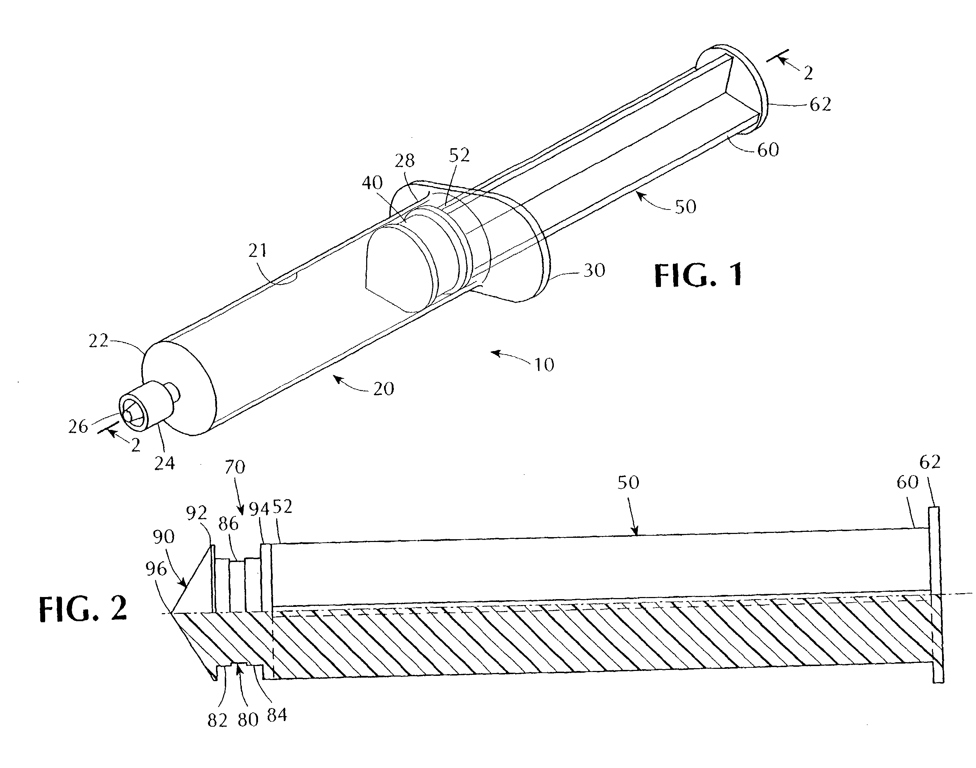

Referring to FIGS. 1 and 2, there is shown a syringe generally designated 10 comprising:
a barrel 20 having inside wall 21, a distal end 22 terminating in a tapered tip 24 which has bore 26 therethrough and proximal end 28 to receive plunger ring 40 to which plunger rod 50 is either removably attached or it may be integral with plunger 40. Plunger rod 50, having distal end 52 and proximal end 60 comprises handle 62 to facilitate exertion of force on plunger ring 40 by plunger rod 50 during operation of the syringe. Disposed about the periphery of proximal end 28 of barrel 20 is finger hub 30 which facilitates holding of barrel 20 during movement of plunger ring 40 by exerting a force on plunger rod 50 in order to move plunger ring 40 in a direction towards the distal end 22 of barrel 20 or in a direction toward the proximal end 28 of barrel 20.

Syringe barrel 20 is made of an inert gas impermeable, substantially transparent material, such as polyethylene, polypropylene, polystyrene, acrylic and methacrylic polymers and preferably of glass.

Plunger ring 40 is made of compressible, elastomeric materials including:
natural rubber;
acrylate-butadiene rubber;
cis-polybutadiene;
chlorobutyl rubber;
chlorinated polyethylene elastomers;
polyalkylene oxide polymers;
ethylene vinyl acetate;
fluorosilicone rubbers;
hexafluoropropylene-vinylidene fluoride-tetrafluoroethylene terpolymers, such as sold under the tradenames of Fluorel and Viton;
butyl rubbers;
polyisobutene, such as sold under the tradename Vistanex;
synthetic polyisoprene rubber;
silicone rubbers;
styrene-butadiene rubbers;
tetrafluoroethylene propylene copolymers; and
thermoplastic-copolyesters.
The durometer of the various elastomeric materials should preferably be in the range of from about 25 to about 80 Shore A.

Referring to FIG. 1, plunger ring 40 attached to plunger rod 50 is slideably received in barrel 20 by manual force exerted on plunge rod 50. Plunger ring 40 forms a sealing but slideable engagement with inside wall 21 of barrel 20.

Referring to FIG. 2, plunger rod 50, having proximal end 60 and distal end 52, further comprises plunger rod tip generally designated at 70. Plunge rod tip 70 having a mushroom shape configuration comprises: a cylindrical shaft 80, the distal end of which is designated at 82 and proximal end is designated at 84, and between the distal end 82 and proximal end 84 of cylindrical shaft 80 there is provided cylindrical recess or groove 86; and cone-shaped head 90 having a circular flange 92, zenith 96 and side 94 connecting flange 92 and zenith 96. Cone-shaped head 90 inside of barrel 20 moves freely without rubbing against inside wall 21 of barrel 20. It is intended to provide a surface area facing the content of the syringe barrel so that, because of its inert thermoplastic composition, no substantial interaction will occur between the content of the barrel and the plunger rod tip 70. Adjacent to proximal end 84 of cylindrical shaft 80, cylindrical shoulder 94 complete the embodiment of plunger rod tip 70. The outside diameter of cylindrical shoulder 94 at least approximates the outside diameter of flange 92 and, preferably is of the same diameter.

Plunger rod tip 70 can be integral with plunger rod 50 as shown in FIG. 2 or it can be removably attached to plunger rod 50 at the distal end 52 thereof.

When plunger rod tip 70 is intended for use in pre-filled syringes or cartridges, it is not permanently attached, that is, it is not integral with plunger rod 50. In this embodiment plunger rod tip is provided with female internal threads in the center portion thereof so that a male threaded plunger rod could be attached thereto.

Cylindrical shaft 80 having distal end 82, proximal end 84 and recess 86 thereon is designed to receive and firmly retain elastomeric plunger ring 40.

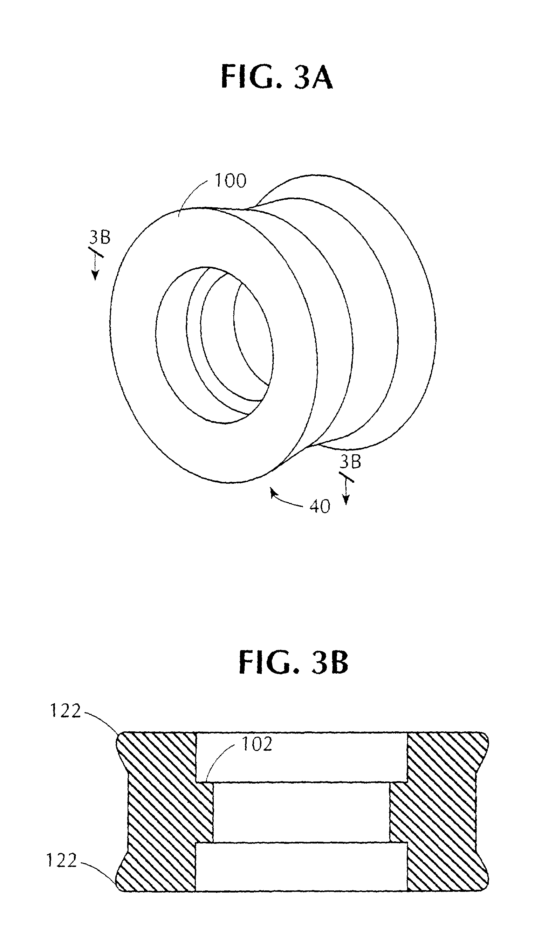

Referring to FIGS. 3A and 3B, plunger ring 40 is of cylindrical shape and is made of elastomeric materials. It comprises an inside wall 100, and an outside wall 120. Inside wall 100 comprises internal ring 102, slightly protruding out from inside wall 100 toward the center of the ring, designed to engage cylindrical recess or groove 86 on cylindrical shaft 80 thereby to form an internal seal between plunger ring 40 and plunger rod tip 70. Internal ring 102 is firmly held by recess or groove 86 so that upon movement of the plunger rod tip 40 in the barrel 20 of the syringe no disengagement can occur between plunger ring 40 and plunger rod tip 70. In all the embodiments of the present invention there must be at least one internal seal formed between said plunger ring and said plunger rod tip 70. However, more than one such seal may be provided in some of the embodiments depending on the intended use of the syringe.

Outside wall 120 of plunger ring 40 comprises at least one, but preferably more, plunger ring rib 122 designed to form secondary seal between it and the inside wall 21 of barrel 20. The configuration of rib or ribs 122 may be horizontal to a plane, or oriented towards the distal or proximal ends of the plunger rod tip 70.

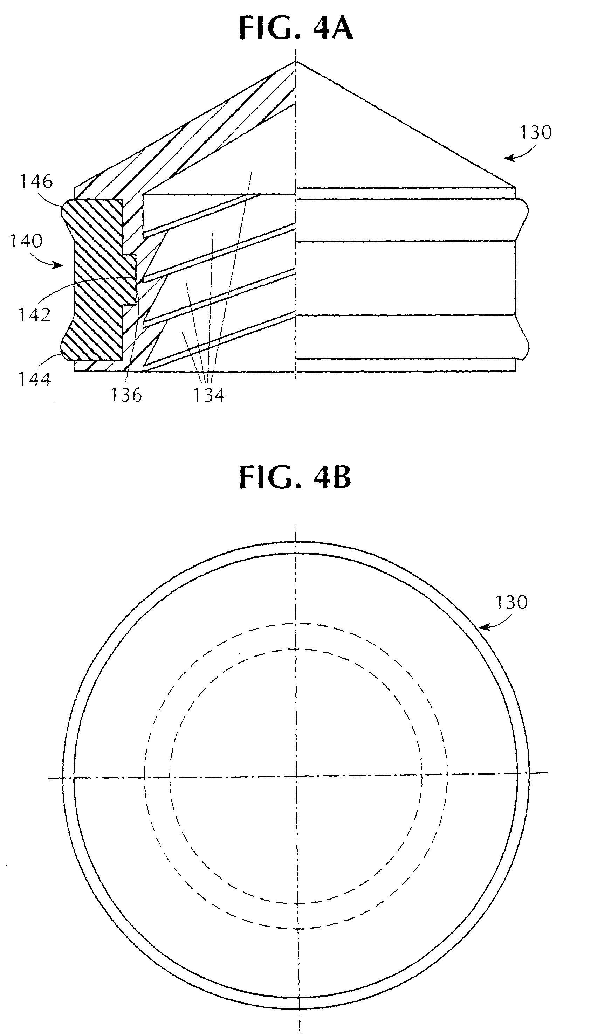

Referring to FIGS. 4A and 4B, there is shown in longitudinal cross-section and top plan view a plunger rod tip 130 with plunger ring 140 thereon, wherein the plunger rod tip 130 has internal female threads 134 therein for attachment to a male-threaded plunger rod.

Plunger ring 140 comprises: internal ring 142 forming an internal seal between it and recess or groove 136 in plunger rod tip 130; and plunger ring ribs 144 and 146 forming secondary seals with the inside wall of a barrel. As shown, two secondary seals are provided by the presence of the horizontal ribs insuring against leakage when the plunger rod tip is moved in the barrel in the proximal or distal direction.

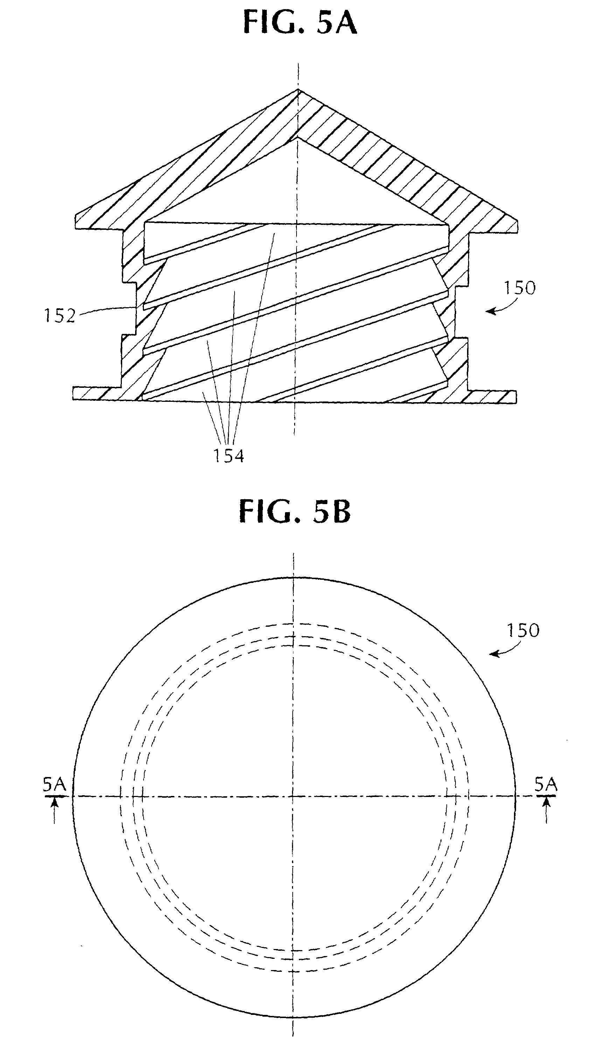

FIGS. 5A and 5B show the longitudinal cross-section and top plan view of a plunger rod tip 150 designed to carry one internal seal plunger ring thereon in recess or groove 152 wherein the plunger rod tip 150 has internal female threads 154 therein for attachment to a male-threaded plunger rod.

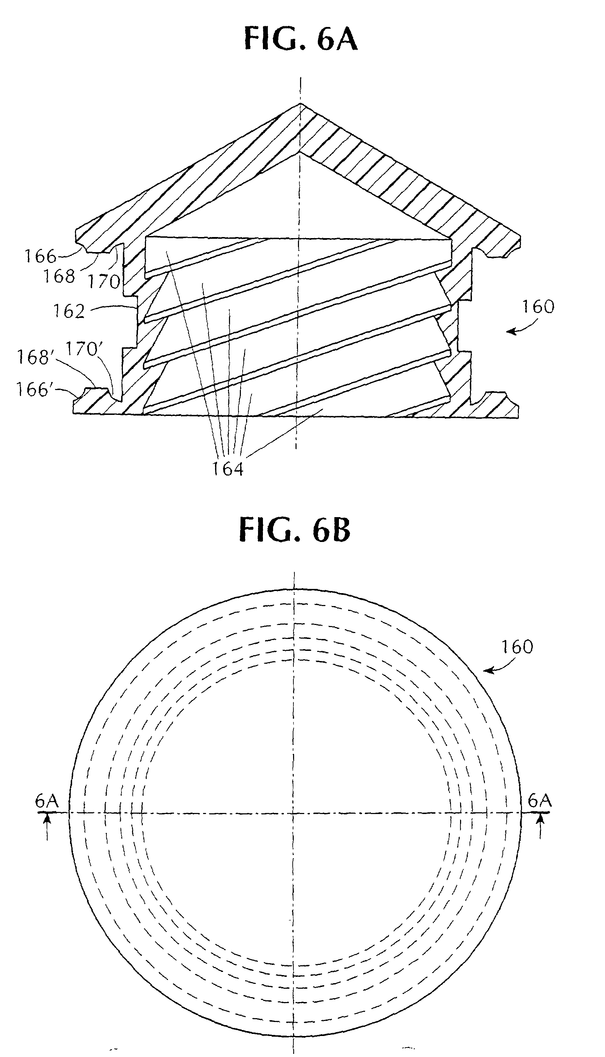

FIGS. 6A and 6B show another longitudinal cross section and top plan view of a plunger rod tip 160 designed to carry one internal seal plunger ring thereon in recess or groove 162 wherein the plunger rod tip 160 has internal female threads 164 thereon for attachment to a male-threaded plunger rod. The plunger rod tip 160 further comprises accentuated contours at 166, 168, 170, 166', 168' and 170' for insuring retention of a matching plunger ring on said plunger rod tip 160.

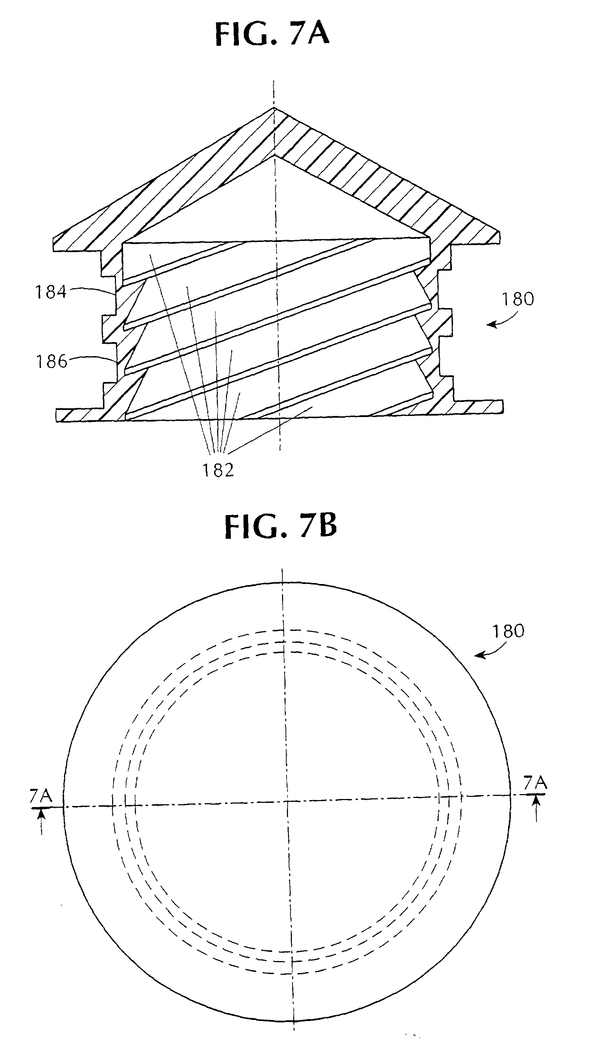

FIGS. 7a and 7B show another longitudinal cross section and top plan view of a plunger rod tip 180 designed to carry two internal seals plunger ring thereon in recess or groove 184 and 186, wherein the plunger rod tip 180 has internal female threads therein for attachment to a male-threaded plunger rod. The presence of two internal seals further insures against leakage at the interface between the plunger rod tip and the plunger ring.

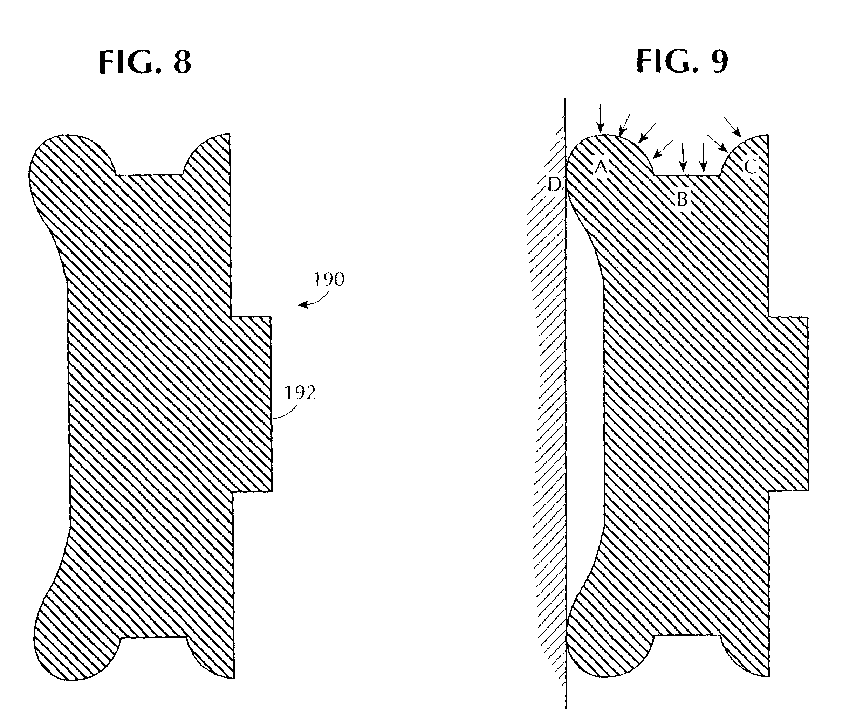

FIG. 8 shows a longitudinal cross-section of a plunger ring 190 having one internal seal provision at 192 with a matching plunger rod tip such as shown in FIGS. 6A and 6B.

FIG. 9 shows the longitudinal cross-section of the plunger ring 190 having one internal seal shown in FIG. 8 and the directions of pressures exerted on the plunger ring in use.

The plunger ring has minimal contact areas with the inside wall of the barrel. The various designs described in the embodiments of the present invention use a hydraulic action to increase the contact area as the pressure increases during use. In FIG. 9, as illustrated, as pressure increases: area A is forced toward the side of the barrel D; area C is forced towards the plunger tip; and area B is compressed. The same hydraulic action works in both directions, i.e. when the plunger rod tip is forced to travel in the distal or proximal directions.

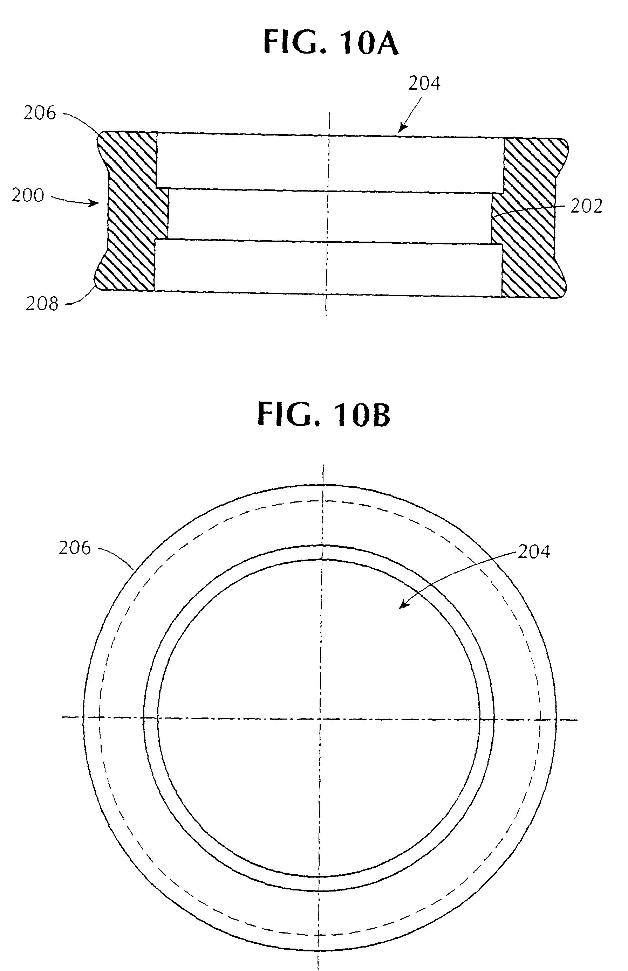

FIGS. 10A and 10B show the longitudinal cross-section and side elevational view of still another plunger ring 200 having one internal seal 202 in place on the plunger rod tip 204 wherein the plunger ring comprises plunger ring rims 206 and 208.

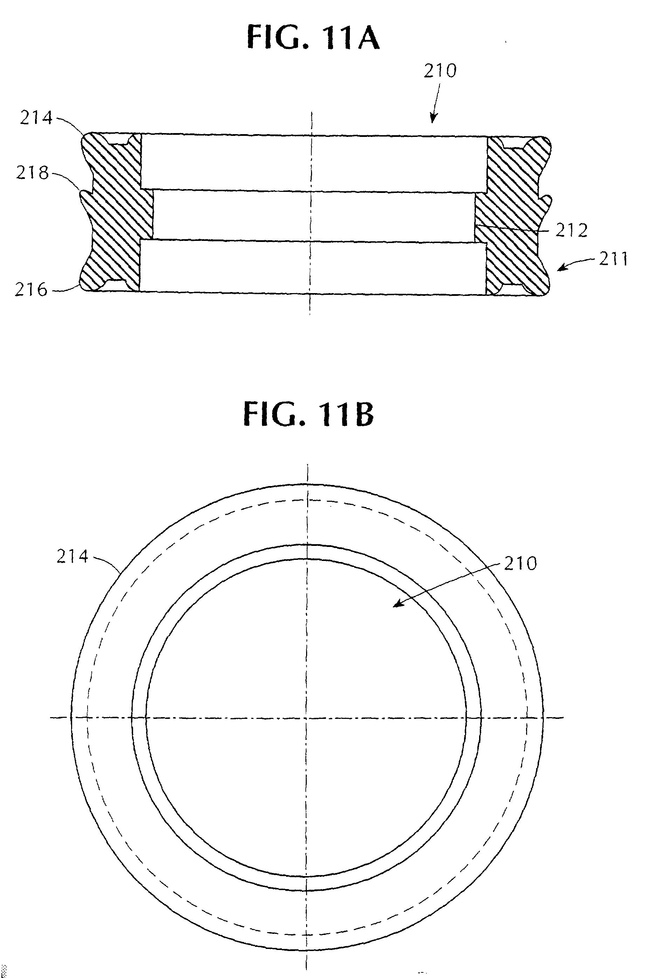

FIGS. 11A and 11B show the longitudinal cross section of still another plunger rod tip 210/plunger ring 211 combination having one internal seal 212 in place on the plunger rod tip, wherein the plunger ring comprises plunger ring rims 214, 216 and 218. These rims are designed to provide leak-proof protection in one direction. As shown in the drawings leak-proof protection is achieved when the plunger ring/plunger tip combination is forced to travel distally, such as when the syringe is used for injection. If the syringe is intended for use in withdrawing biological fluid from a patient, the orientation of the plunger ring rib/plunger rod tip combination would be reversed 180°.

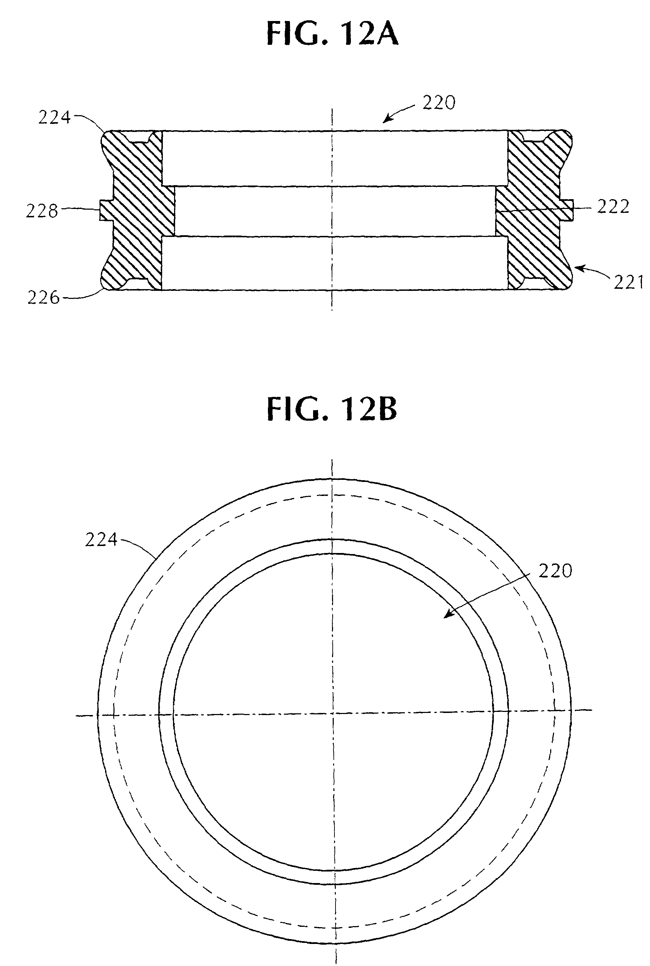

FIGS. 12A and 12B show a longitudinal cross-section and side elevational view of still another plunger rod tip 220/plunger ring 221 combination having an internal seal 222 in place on the plunger rod tip, wherein the plunger ring comprises plunger ring rims 224, 226 and 228. This embodiment of the present invention is designed to provide leak-proof protection in both the proximal and distal directions.

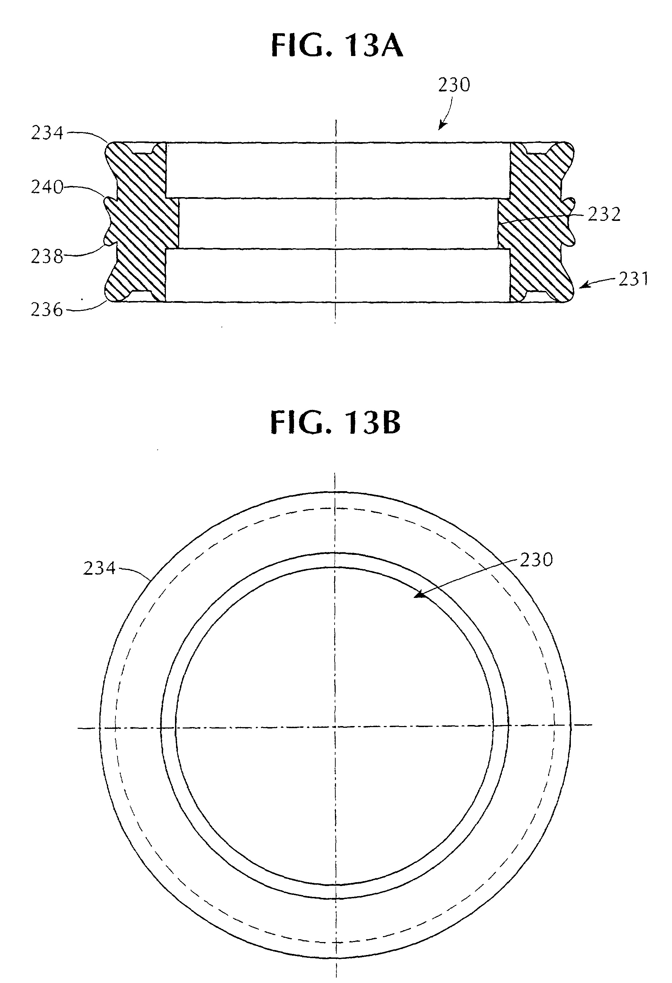

FIGS. 13A and 13B show a longitudinal cross-section and side elevational view of still another plunger rod tip 230 having an internal seal 232 in place on the plunger rod tip/plunger ring 231 combination, wherein the plunger ring comprises plunger ring rims 234, 236 and 238 and 240. This embodiment of the present invention is also designed to provide leak-proof protection in both the proximal and distal directions.

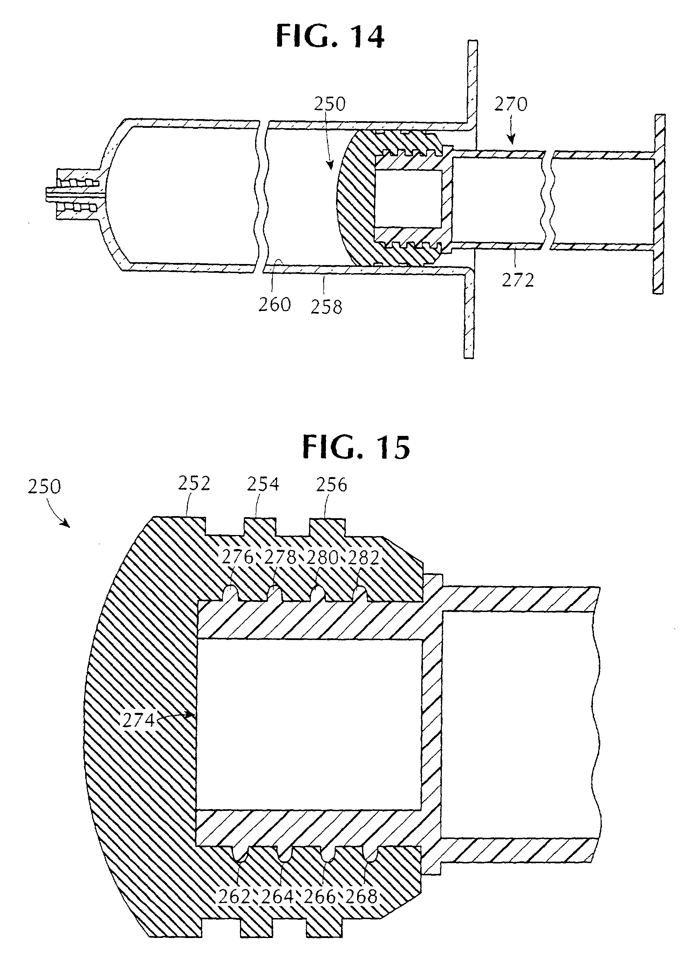

FIGS. 14 shows a longitudinal fragmentary cross-section of a typical prior art syringe, plunger and plunger rod; and FIG. 15 show an enlarged fragmentary cross-section thereof. Plunger 250, made of elastomeric material, comprises external plunger rims 252, 254 and 256 which are designed to provide a leak-proof seal between the plunger and the inside wall 260 of barrel 258. Plunger 250 is provided with female threads 262, 264, 266 and 268 to receive a male-threaded plunger rod. Plunger rod 270 of this prior art embodiment comprises shank 272 and plunger rod head 274 which is provided with male threads 276, 278, 280 and 282 to engage female threads 262, 264, 266 and 268. It is apparent that the content of the barrel, such as a medical solution, interfaces with the elastomeric plunger thereby allowing undesirable interaction therebetween. Such interaction may result in the dissolution of at least small amounts of chemicals present in the elastomeric material which then taints the medical fluids.

An embodiment of the present invention shown in FIG. 1 was comparatively tested against a corresponding prior art device illustrated in cross-section in FIGS. 14 and 15. The summary of the test results expressed as averages in N (lbs) is shown in Table I.

**TABLE I**

| | **Present Invention** | **Prior Art** |
|---|---|---|
| Breakaway Force With Silicone | 0.67 N (0.15 lbs) | 19.13 N (4.3 lbs) |
| Breakaway Force Without Silicone | 2.71 N (0.61 lbs) | 75.17 N (16.9 lbs) |
| Running Force With Silicone | 0.76 N (0.17 lbs) | 11.12 N (2.5 lbs) |
| Running Force Without Silicone | 2.69 N (0.60 lbs) | 51.60 N (11.6 lbs) |
| Leakage | None | Some observed between plunger ribs |
| Test to Failer (Breakage) | 400.3 N (90.0 lbs) | 382.6 N (86.0 lbs) |

In addition to the extremely low breakaway and running forces shown in Table I, contact between the medical fluid in the syringe barrel and the elastomeric (rubber) material constituting a portion of the plunger of the present invention was reduced about 80%, thereby reducing the tendency of the presence of elastomeric exudate in the medical fluid.

Description of the details of the comparative testing follows.

Testing was conducted using three pre-filled syringes for each test. Measurements were made with a standard Test Gauge at five points in the barrel of the syringes as the plunger was forced to travel down towards the distal end of the syringe:
Test Point 1 was at the proximal end of the barrel;
Test Point 2 was at about 25% down from the proximal end of the barrel;
Test Point 3 was at about 50% down from the proximal end of the barrel;
Test Point 4 was at about 75% down from the proximal end of the barrel; and
Test point 5 was at the distal end of the barrel, close to where the barrel tapers to form the tip.

The result of the testing of the syringes are shown in the following Figures.

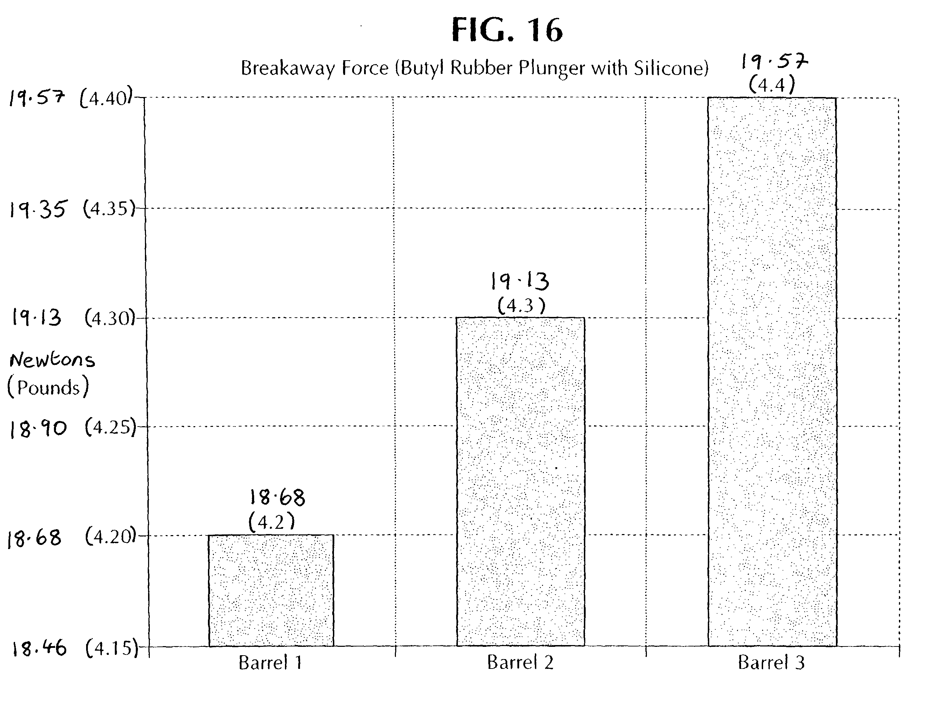

FIG. 16 shows a graph of breakaway forces between the inside walls of three barrels and butyl rubber plungers with silicone of the prior art, wherein Barrel 1, Barrel 2 and Barrel 3 show a breakaway force of 18.68, 19.13 and 19.57 N (4.2, 4.3 and 4.4 pounds), respectively.

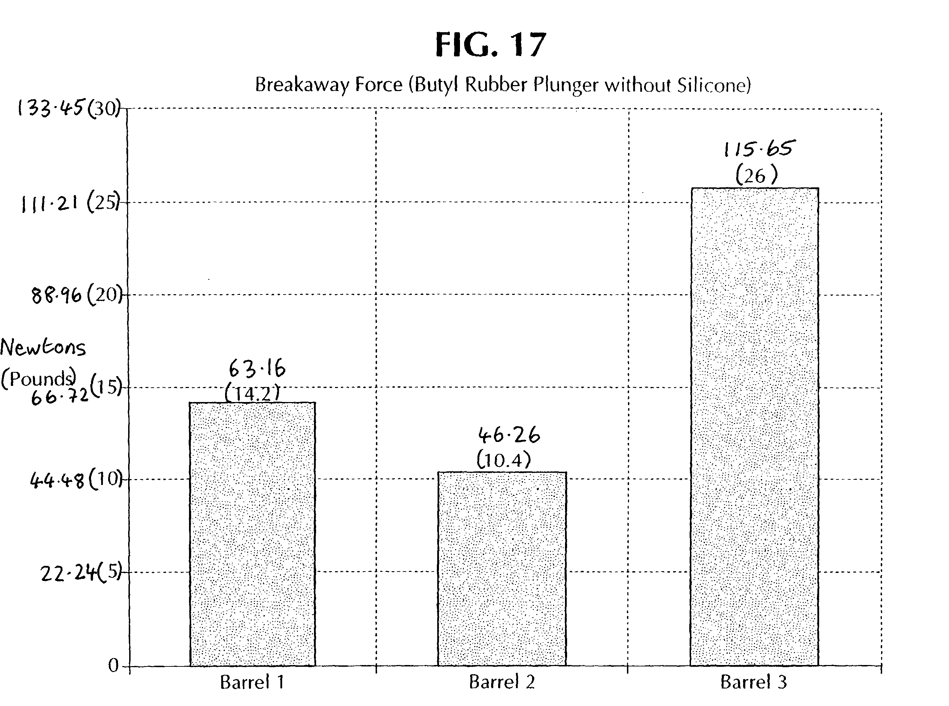

FIG. 17 shows a graph of breakaway forces between the inside walls of three barrels and butyl rubber plungers without silicone of the prior art, wherein Barrel 1, Barrel 2 and Barrel 3 show a breakaway force of 63.16, 46.26 and 115.65 N (14.2, 10.4 and 26 pounds), respectively. It is apparent that without the use of silicone the breakaway forces have substantially increased.

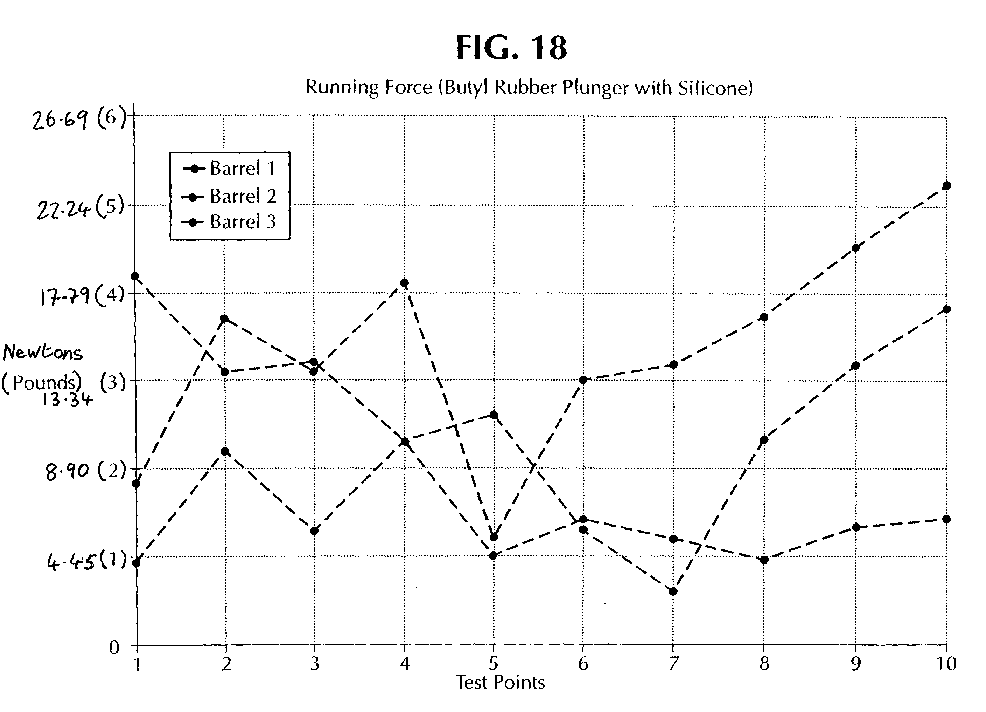

FIG. 18 shows a graph of running forces between the inside walls of three barrels and butyl rubber plungers with silicone of the prior art, wherein the running forces are shown to vary from about 3.34 N to about 23.1 N (about 0.75 pounds to about 5.2 pounds).

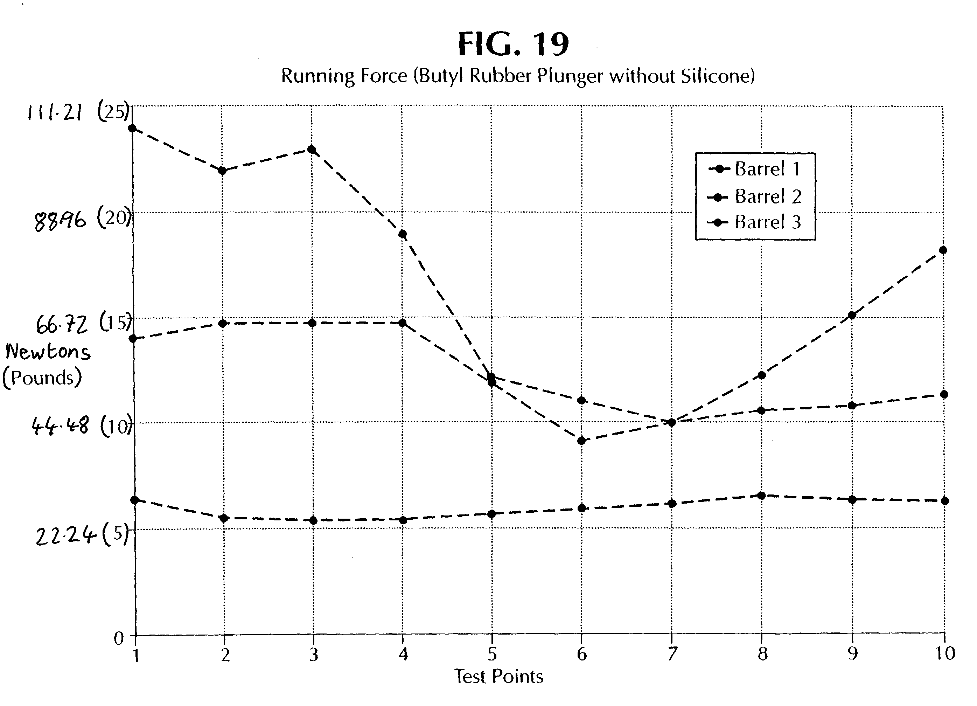

FIG. 19 shows a graph of running forces between the inside walls of three barrels and butyl rubber plungers without silicone of the prior art, wherein the running forces are shown to vary from about 53.38 N to about 106.76 N (about 12 pounds to about 24 pounds). Again, it is apparent that V without the use of silicone the running forces have substantially increased, similarly to the breakaway forces shown in FIGS. 16 and 17.

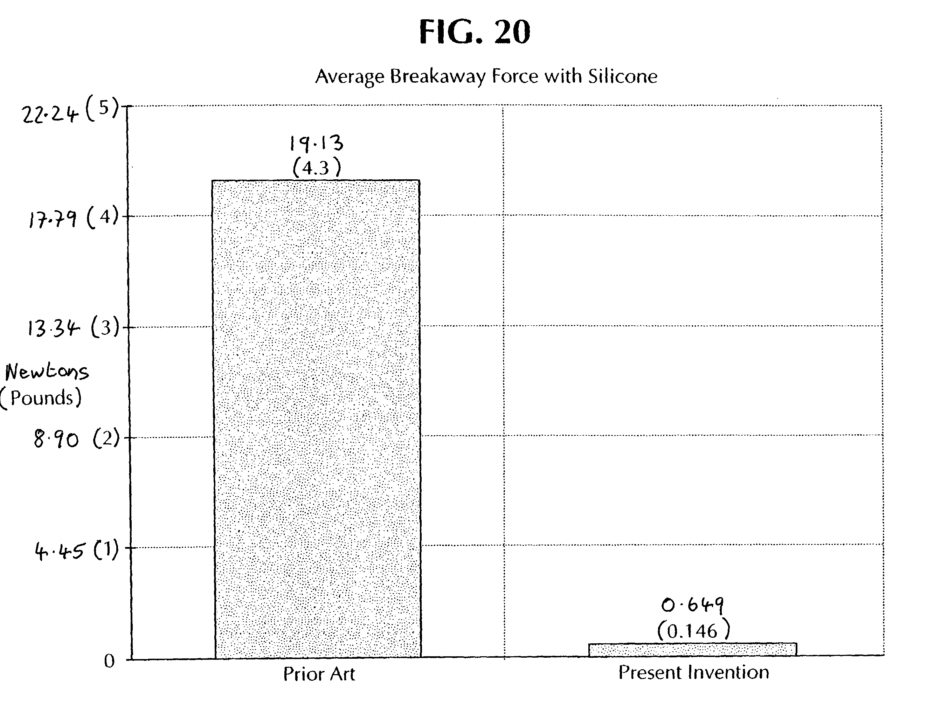

FIG. 20 shows a graph of average breakaway forces between the inside walls of the barrels and the butyl rubber plungers with silicone of the prior art, and the average breakaway forces between the inside walls of the barrels and the plunger rod tip/plunger ring combination with silicone of the present invention.

It is apparent from the graphs that the average breakaway force of the prior art device is many-fold larger than that of the present invention.

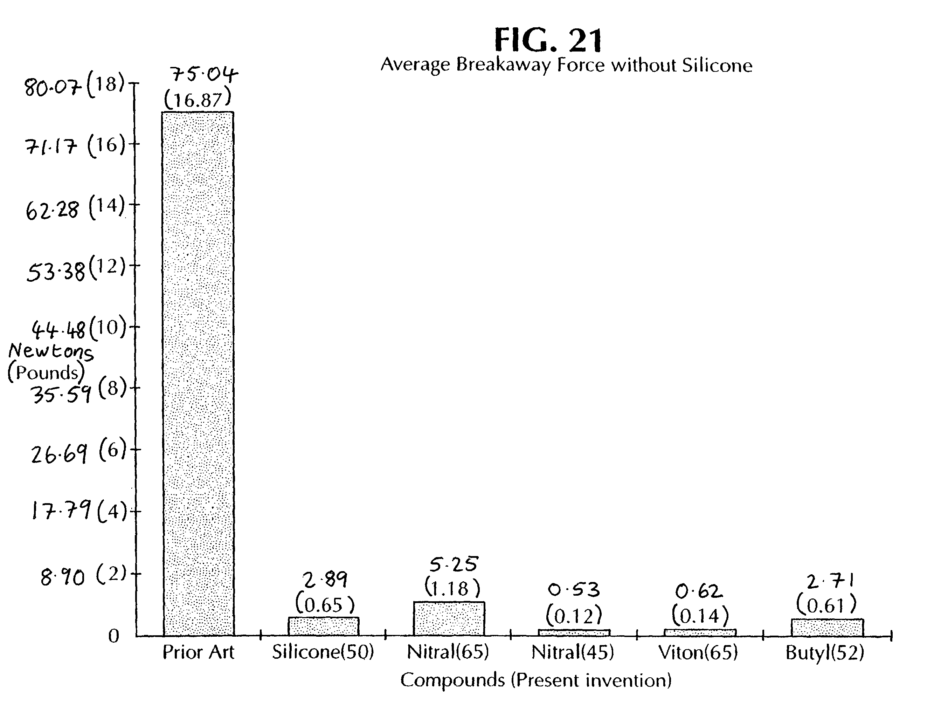

FIG. 21 shows a graph of average breakaway forces between the inside walls of the barrels and the butyl rubber plungers without silicone of the prior art, and the average breakaway forces between the inside walls of the barrels and the plunger rod tip/plunger ring combination without silicone of the present invention wherein the plunger ring is made of Silicone(50), Nitrial(65), Nitrial(45), Viton(65) or Butyl(52) rubbers the average breakaway force for each of which is shown in the graph.

The breakaway forces of the prior art device is at least 25, 14, 140, 120 and 27 times larger than the breakaway forces of the present invention respectively, using the rubber materials from which the plunger rings are made.

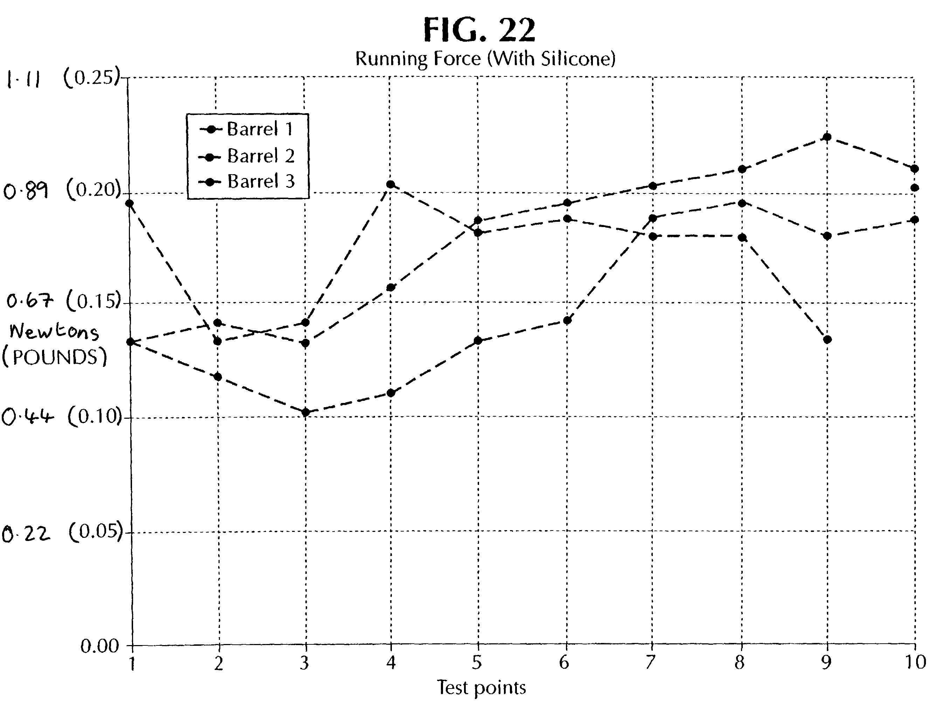

FIG. 22 shows a graph of running forces between the inside wall of the barrel and the plunger rod tip/butyl rubber plunger ring combination with silicone of the present invention showing the result for each of three barrels.

The running forces for Barrel 1 range of from about 0.44 N to about 0.80 N (about 0.1 lbs to about 0.18 lbs), the running forces for Barrel 2 range of from about 0.58 N to about 0.93 N (about 0.13 lbs to about 0.21 lbs), and the running forces for Barrel 3 range of from about 0.58 N to about 1.02 N (about 0.13 lbs to about 0.23 lbs).

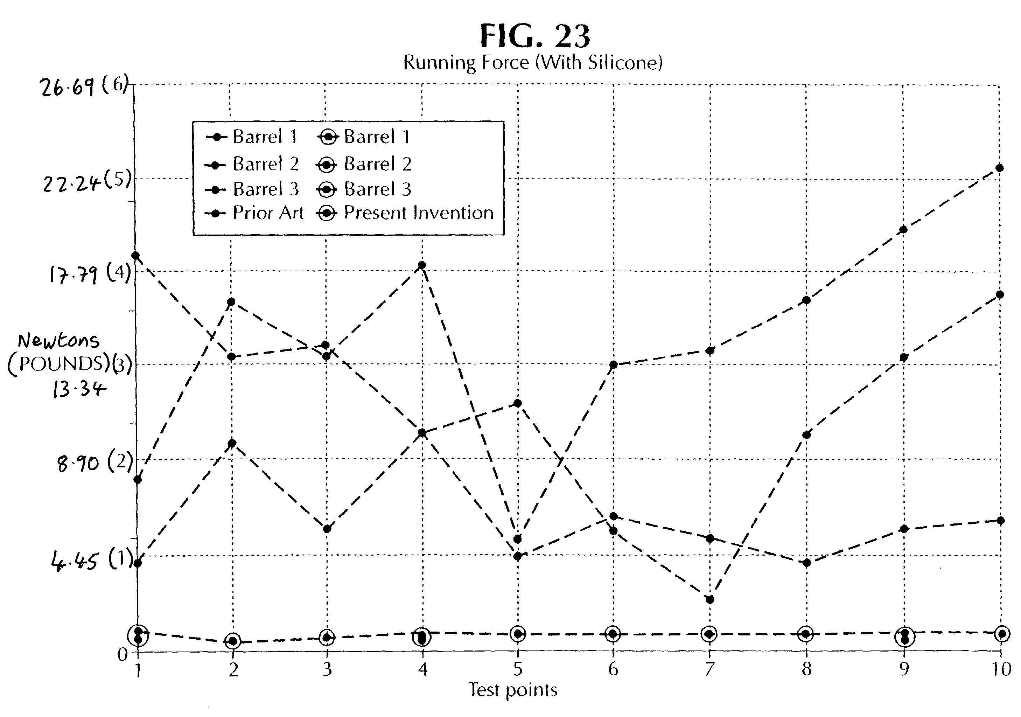

FIG. 23 shows a graph of running forces between the inside wall of the barrel and the plunger rod of the prior art made of butyl rubber with silicone, versus a graph a running forces between the inside wall of the barrel and the plunger rod tip/plunger ring combination made of butyl rubber with silicone of the present invention.

As shown, the running forces of the prior art device are in the range of from about 2.69 N to about 23.13 N (about 0.60 lbs to about 5.2 lbs), while the running forces of the present invention is in the range of about 1.11 N (about 0.25 lbs).

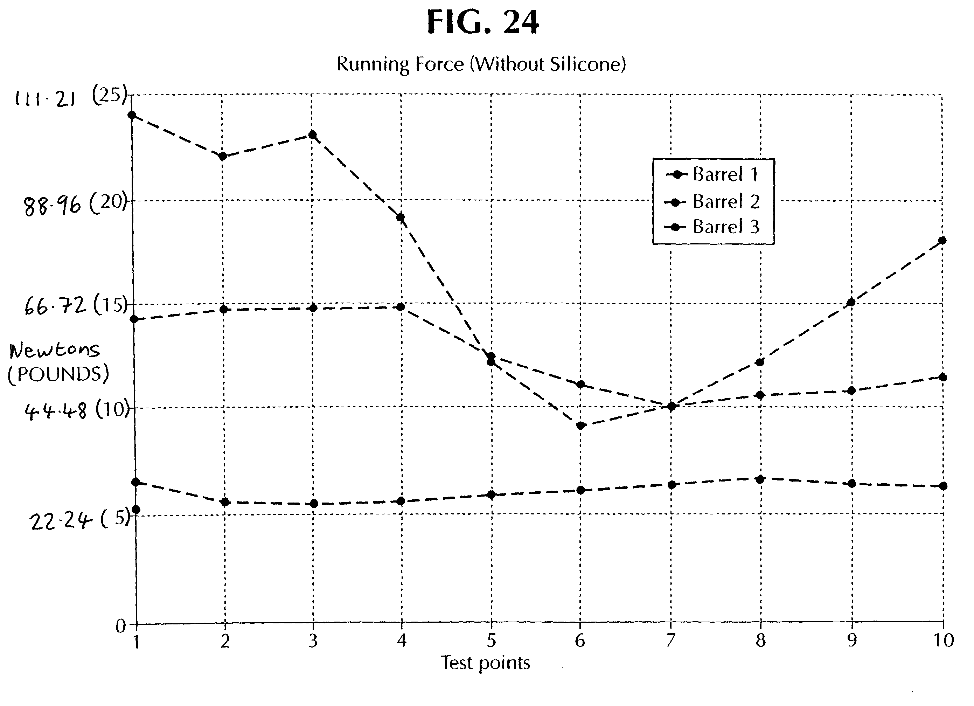

FIG. 24 shows a graph of running forces between the inside wall of the barrel and the plunger made of butyl rubber without silicone of the prior art for Barrels 1, 2 and 3.

For Barrel 1, the running forces range from about 24.47 N to about 26.69 N (about 5.5 lbs to about 6.0 lbs), for Barrel 2, the running forces range from about 31.14 N to about 53.38 N (about 7.0 lbs to about 12.0 lbs), and for Barrel 3 the running forces range from about 106.76 N to about 80.07 N (about 24.0 lbs to about 18.0 lbs).

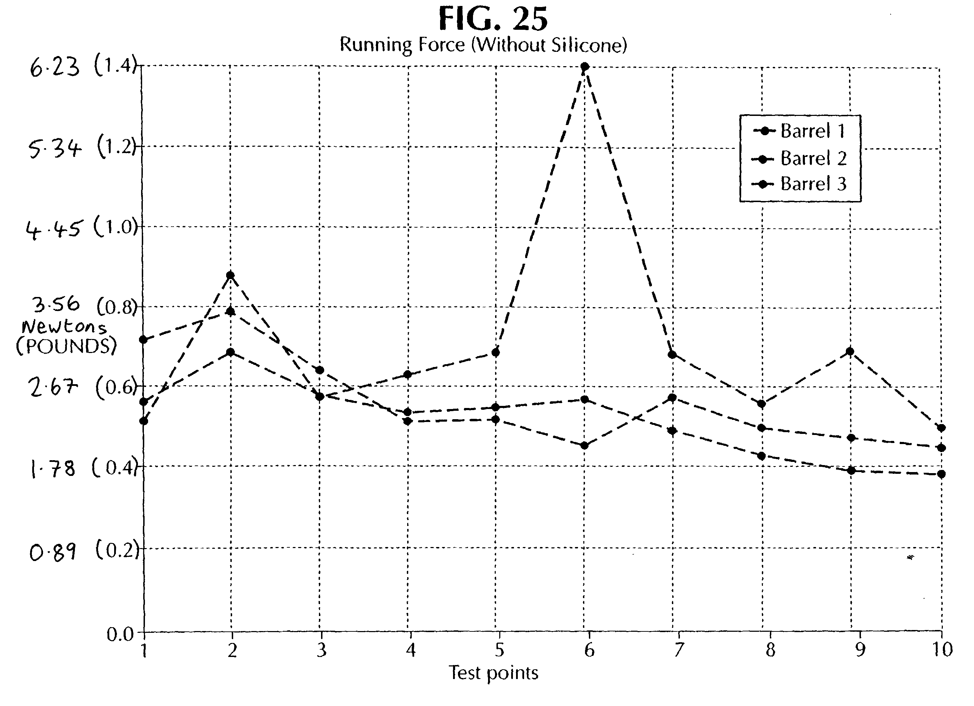

FIG. 25 shows a graph of running forces between the inside wall of the barrel and the plunger rod tip/plunger ring combination without silicone of the present invention for Barrels 1, 2 and 3.

For Barrel 1, the running forces range from about 2.22 N to about 6.23 N (about 0.50 lbs to about 1.40 lbs), for Barrel 2, the running forces range from about 1.78 N to about 2.89 N (about 0.40 lbs to about 0.65 lbs), and the running forces for Barrel 3 range from about 3.56 N to about 2.00 N (about 0.80 lbs to about 0.45 lbs).

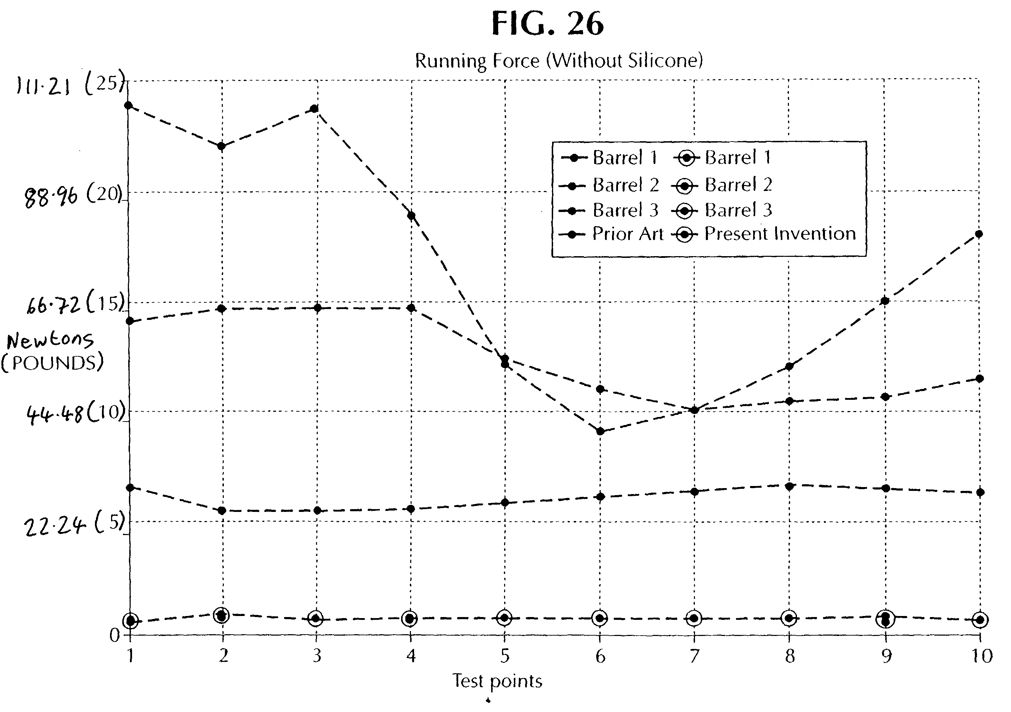

FIG. 26 shows a graph of running forces between the inside wall of the barrel and the plunger made of butyl rubber without silicone of the prior art for Barrels 1, 2 and 3, versus a graph of running forces between the inside wall of the barrel and the plunger rod tip/plunger ring combination made of butyl rubber without silicone of the present invention.

It is apparent from the graphs that the running forces existing in the prior art without the use of silicone are large, while the running forces existing in the present invention without silicone are very low.

For the prior art the running forces for Barrel 1 range of from about 24.47 N to about 26.69 N (about 5.5 lbs to about 6.0 lbs), for Barrel 2 the running forces range of from about 40.03 N to about 51.15 N (about 9.0 lbs to about 11.5 lbs), and for Barrel 3 the running forces range of from about 104.53 N to about 80.07 N (about 23.5 lbs to about 18.0 lbs).

| **LIST OF REFERENCE NUMBERS USED** | |
|---|---|
| Syringe (generally designated) | 10 |
| Barrel | 20 |
| Inside wall of barrel | 21 |
| Distal end of barrel | 22 |
| Tapered tip of barrel at distal end | 24 |
| Bore through tip of barrel | 26 |
| Proximal end of barrel | 28 |
| Finger hub on proximal end of barrel | 30 |
| Plunger ring (generally designated) | 40 |
| Plunger rod (generally designated) | 50 |
| Distal end of plunger rod | 52 |
| Proximal end of plunger rod | 60 |
| Handle of plunger rod | 62 |
| Plunger rod tip (generally designated) | 70 |
| Cylindrical shaft of plunger rod tip (generally designated) | 80 |
| Distal end of plunger rod tip | 82 |
| Proximal end of plunger rod tip | 84 |
| Cylindrical recess or groove in plunger rod tip | 86 |
| Cone-shaped head of plunger rod tip (generally designated) | 90 |
| Flange of cone-shaped head of plunger rod tip | 92 |
| Cylindrical shoulder on plunger rod tip | 94 |
| Inside wall of plunger ring | 100 |
| Outside wall of plunger ring | 120 |
| Internal ring on inside wall of plunger ring | 102 |
| Plunger rod tip (of another embodiment, generally designated) | 130 |
| Internal female threads on plunger rod tip | 134 |
| Plunger ring (generally designated) | 140 |
| Internal ring on inside wall of plunger ring | 142 |
| Plunger ring ribs | 144,146 |
| Plunger rod tip (of still another embodiment, generally designated) | 150 |
| Recess or groove in plunger rod tip | 152 |
| Internal female threads on plunger rod tip | 154 |
| Plunger rod tip (of still another embodiment, generally designated) | 160 |
| Recess groove in plunger rod tip | 162 |
| Internal female threads on plunger rod tip | 164 |
| Accentuated contours on plunger rod tip | 166,168,170,166', |
| | 168',170' |
| Plunger rod tip (of still another embodiment, generally designated) | 180 |
| Recess or groove in plunger rod tip | 184,186 |
| Plunger ring (of still another embodiment, generally designated) | 190 |
| Internal seal on plunger ring | 192 |
| Plunger ring (of still another embodiment, generally designated) | 200 |
| Internal seal on plunger ring | 202 |
| Plunger rod tip | 204 |
| Plunger ring ribs | 206,208 |
| Plunger rod tip (of still another embodiment, generally designated) | 210 |
| Plunger ring (generally designated) | 211 |
| Internal seal on plunger rod tip | 212 |
| Plunger ring ribs | 214, 216, 218 |
| Plunger rod tip (of still another embodiment, generally designated) | 220 |
| Plunger ring | 221 |
| Internal seal on plunger rod tip | 222 |
| Plunger ring ribs | 224, 226, 228 |
| Plunger rod tip (of still another embodiment, generally designated) | 230 |
| Plunger ring | 231 |
| Internal seal on plunger rod tip | 232 |
| Plunger ring ribs | 234, 236, 238, 240 |
| | |
| **Prior Art** | |
| Plunger | 250 |
| External plunger rims | 252, 254, 256 |
| Inside wall of barrel | 258 |
| Female threads on plunger | 262, 264, 266, 268 |
| Plunger rod | 270 |
| Shenk of plunger rod | 272 |
| Head of plunger rod | 274 |
| Male threads in plunger rod head | 276, 278, 280, 282 |

Various modifications of the present invention disclosed will become apparent. This invention is intended to include such modifications to be limited only by the scope of the claims.

## Claims

1. A syringe (10), optionally prefilled, designed for injecting a fluid into a site or withdrawing a fluid from a site comprising:
(a) a syringe barrel (20) having an inner surface (21) defining a cylindrical chamber for retaining a fluid therein;
(b) a plunger slideably mounted in said barrel (20) and positioned close to the proximal end (28) of the barrel for injecting a fluid into a site, or positioned close to the distal end (22) of the barrel for withdrawing fluid from a site,
**characterised in that**, said plunger comprises a rigid plunger rod tip (70, 130, 150, 160, 180, 204, 210, 220, 230) and an elastomeric plunger ring (40, 140, 190, 200, 211, 221, 231), wherein said elastomeric plunger ring (40, 140, 190, 200, 211, 221, 231) has an inside wall (100) and an outside wall (120),
(1) said inside wall (100) comprises at least one internal ring (102) slightly protruding out towards the centre of the ring designed to engage a recess or groove (86, 152, 162, 184, 186) in a cylindrical shaft (80) of said rigid plunger rod tip (70, 130, 150, 160, 180, 204, 210, 220, 230) and form a non-slideable internal seal therewith;
(2) said outside wall (120) comprises at least one plunger ring rib (144, 146, 206, 208, 214, 216, 218, 224, 226, 228, 234, 236, 238, 240) designed to form a secondary slideable seal with the inside wall (21) of said barrel (20).

2. A syringe (10) according to claim 1, wherein said rigid plunger rod tip (70, 130, 150, 160, 180, 204, 210, 220, 230) has a mushroom-shape configuration comprising:
(i) the cylindrical shaft (80) having a distal end (82) and a proximal end (84), and the recess or groove (86, 152, 162, 184, 186), between said distal end (82) and said proximal end (84), designed to receive said elastomeric plunger ring (40, 140, 190, 200, 211, 221, 231) and forming non-slideable internal seal therewith;
(ii) a cone-shaped head (90) integral with said cylindrical shaft (80), said cone-shaped head (90) having an outside diameter slightly smaller than the inside diameter of said barrel (20) allowing free movement of said cone-shaped head (90) in said barrel (20);
(iii) said elastomeric plunger ring (40, 140, 190, 200, 211, 221, 231) mounted on said cylindrical shaft (80) of said rigid plunger rod tip (70, 130, 150, 160, 180, 204, 210, 220, 230).

3. A syringe (10) according to claim 2, wherein said cone-shaped head (ii) (90) has a circular flange (92) on the proximal end thereof, a zenith (96) at the centre on the distal end thereof and side connecting said flange (92) and said zenith (96).

4. A syringe (10) according to claims 1-3, wherein said plunger rod tip (70, 130, 150, 160, 180, 204, 210, 220, 230) is made of polymeric material; said syringe barrel (20) is made of glass or polymeric material, said barrel (20) having:
(I) a distal end terminating in a tapered tip (24) and having a bore (26) thereto to which an injection needle or a connector equipped with tubing conduit can be attached; and
(II) a proximal end (28) for receiving a plunger.

5. A syringe (10) according to claims 1-4, wherein said rigid plunger rod tip (70, 130, 150, 160, 180, 204, 210, 220, 230) further comprises an integral plunger rod (50) at its proximal end thereof.

6. A syringe (10) according to claims 1-5, wherein said plunger rod tip further comprises attachment means at the proximal end thereof.

7. A syringe according to claim 6, wherein said attachment means comprises female threads (164) in said plunger rod tip (70, 130, 150, 160, 180, 204, 210, 220, 230).

8. A syringe (10) according to claims 1-7, wherein said plunger rod tip (70, 130, 150, 160, 180, 204, 210, 220, 230) comprises two or more recess or grooves (86, 152, 162, 184, 186) to receive said elastomeric plunger ring to form non-slidably seals thereon.

9. A syringe (10) according to claim 8, wherein said elastomeric plunger ring comprises two or more plunger ring ribs (144, 146, 206, 208, 214, 216, 218, 224, 226, 228, 234, 236, 238, 240) thereon to form secondary slideable seals with the inside wall of said barrel.

10. A syringe (10) according to claims 1-9, wherein said plunger ring rib is horizontally oriented towards the inside wall of said barrel.

11. A syringe according to claims 1-9, wherein said plunger ring rib is oriented towards the distal end of the barrel (22).

12. A syringe according to claims 1-9, wherein said plunger ring rib is oriented towards the proximal end of the barrel (28).

13. A syringe according to claims 1-9, wherein at least one of said plunger ring ribs is oriented towards the distal end (22) of the barrel and at least one of said plunger ring ribs is oriented towards the proximal end (28) of the barrel.

14. A syringe according to claims 1-9, comprising three plunger ring ribs one of which is oriented horizontally, the second of which is oriented towards the distal end of the barrel and the third of which is oriented towards the proximal end of the barrel.

15. A syringe according to claims 1-14, wherein said syringe barrel is made of an inert gas impermeable material selected from the group consisting of polyethylene, polypropylene, polystyrenes, acrylic polymers and methacrylic polymers.

16. A syringe according to claims 1-15, wherein said plunger rod tip is made of a thermoplastic material.

17. A syringe according to claims 1-16, wherein said plunger rod tip is made of a material selected from the group consisting of polyethylene, polypropylene, polystyrenes, acrylic polymers and methacrylic polymers.

18. A syringe according to claims 1-17, wherein said plunger ring is made of a compressible, elastomeric material selected from the group consisting of natural and synthetic rubbers.

19. A syringe according to claims 1-18, wherein said elastomeric material is selected from the group consisting of:
natural rubber;
acrylate-butadiene rubber;
cis-polybutadiene;
chlorobutyl rubber;
chlorinated polyethylene elastomers;
polyalkylene oxide polymers;
ethylene vinyl acetate;
fluorosilicone rubbers;
hexafluoropropylene-vinylidene;
tetrafluoroethylene terpolymers;
butyl rubbers;
polyisobutene;
synthetic polyisoprene rubber;
silicone rubbers;
styrene-butadiene rubbers;
tetrafluoroethylene propylene copolymers; and
thermoplastic-copolyesters.

20. A syringe according to claims 1-19, wherein said fluid is a medical fluid.

21. A syringe according to claim 20, wherein said medical fluid is diagnostic contrast media.

22. A cartridge (10), optionally prefilled, designed for injecting a fluid into a site or withdrawing a fluid from a site comprising:
(a) a cartridge barrel (20) of having an inner surface (21) defining a cylindrical chamber for retaining a fluid therein;
(b) a plunger slideably mounted in said barrel (20) and positioned close to the proximal end (28) of the barrel for injecting a fluid into a site,
**characterised in that**, said plunger comprises a rigid plunger rod tip (70, 130, 150, 160, 180, 204, 210, 220, 230) and an elastomeric plunger ring (40, 140, 190, 200, 211, 221, 231), wherein said elastomeric plunger ring (40, 140, 190, 200, 211, 211, 231) has an inside wall (100) and an outside wall (120),
(1) said inside wall (100) comprises at least one internal ring (102) slightly protruding out towards the centre of the ring designed to engage a recess or groove (86, 152, 162, 184, 186), in a cylindrical shaft (80) of said rigid plunger rod tip and form a non-slideable internal seal therewith;
(2) said outside wall (120) comprises at least one plunger ring rib (144, 146, 206, 208, 214, 216, 218, 224, 226, 228, 236, 238, 240) designed to form a secondary slideable seal with the inside wall (21) of said barrel (20).
.

23. A cartridge (10) according to claim 22, wherein said rigid plunger rod tip (70, 130, 150, 160, 180, 204, 210, 220, 230) has a mushroom-shape configuration comprising:
(i) the cylindrical shaft (80) having a distal end (82) and a proximal end (84), and the recess or groove (86, 152, 162, 184, 186), between said distal end and said proximal end, designed to receive said elastomeric plunger ring (40, 140, 190, 200, 211, 221, 231) and forming the non-slideable internal seal therewith;
(ii) a cone-shaped head (90) integral with said cylindrical shaft (80), said cone-shaped head having an outside diameter slightly smaller than the inside diameter of said barrel (20) allowing free movement of said cone-shaped head (90) in said barrel (20);
(iii) said elastomeric plunger ring (40, 140, 190, 200, 211, 221, 231) mounted on said cylindrical shaft (80) of said rigid plunger rod tip.

24. A cartridge (10) according to claim 23, wherein said cone-shaped head (ii)(90) has a circular flange (92) on the proximal end thereof, a zenith (96) at the centre on the distal end thereof and side connecting said flange (92) and said zenith (96).

25. A cartridge (10) according to claims 22-24, wherein said plunger rod tip (70, 130, 150, 160, 180, 204, 210, 220, 230) is made of polymeric material; said barrel (20) is made of glass or polymeric material, said barrel having:
(I) a distal end terminating in a tapered tip (24) and having a bore (26) thereto to which an injection needle or a connector equipped with tubing conduit can be attached; and
(II) a proximal end (28) for receiving a plunger.

26. A cartridge (10) according to claims 22-25, wherein said rigid plunger rod tip further comprises an integral plunger rod (50) at its proximal end thereof.

27. A cartridge according to claims 22-26, wherein said plunger rod tip further comprises attachment means at the proximal end thereof.

28. A cartridge according to claim 27, wherein said attachment means comprises female threads (164) in said plunger rod tip.

29. A cartridge according to claims 22-28, wherein said plunger rod tip comprises two or more recess or grooves to receive said elastomeric plunger ring to form non-slidably seals thereon.

30. A cartridge according to claim 29, wherein said elastomeric plunger ring comprises two or more plunger ring ribs thereon to form secondary slideable seals with the inside wall of said barrel.

31. A cartridge according to claims 22-30, wherein said plunger ring rib is horizontally oriented towards the inside wall of said barrel.

32. A cartridge according to claims 22-30, wherein said plunger ring rib is oriented towards the distal end of the barrel.

33. A cartridge according to claims 22-30, wherein at least one of said plunger ring ribs is oriented towards the distal end of the barrel and at least one of said plunger ring ribs is oriented towards the proximal end of the barrel.

34. A cartridge according to claims 22-30, comprising three plunger ring ribs one of which is oriented horizontally, the second of which is oriented toward the distal end of the barrel and the third of which is oriented towards the proximal end of the barrel.

35. A cartridge according to claims 22-34, wherein said cartridge barrel is made of an inert gas impermeable material selected from the group consisting of polyethylene, polypropylene, polystyrenes, acrylic polymers and methacrylic polymers.

36. A cartridge according to claims 22-35, wherein said plunger rod tip is made of a thermoplastic material.

37. A cartridge according to claims 22-36, wherein said plunger rod tip is made of a material selected from the group consisting of polyethylene, polypropylene, polystyrenes, acrylic polymers and methacrylic polymers.

38. A cartridge according to claims 22-37, wherein said plunger ring is made of a compressible, elastomeric material selected from the group consisting of natural and synthetic rubbers.

39. A cartridge according to claims 22-38, wherein said elastomeric material is selected from the group consisting of:
natural rubber;
acrylate-butadiene rubber;
cis-polybutadiene;
chlorobutyl rubber;
chlorinated polyethylene elastomers;
polyalkylene oxide polymers;
ethylene vinyl acetate;
fluorosilicone rubbers;
hexafluoropropylene-vinylidene;
tetrafluoroethylene terpolymers;
butyl rubbers;
polyisobutene;
synthetic polyisoprene rubber;
silicone rubbers;
styrene-butadiene rubbers;
tetrafluoroethylene propylene copolymers; and
thermoplastic-copolyesters.

40. A cartridge according to claims 22-39, wherein said fluid is an injectable medical fluid.

41. A cartridge according to claim 40, wherein said injectable medical fluid is diagnostic contrast media.

## Patentansprüche

1. Eine Spritze (10), optional vorbefüllt, die zur Injektion einer Flüssigkeit oder zum Abziehen von Flüssigkeit dient und sich wie folgt zusammensetzt:
a.) einem Spritzenzylinder (20) mit einer Innenfläche (21), die eine zylindrische Kammer zur Aufnahme von Flüssigkeit definiert;
b.) einem Kolben, der beweglich in besagtem Spritzenzylinder (20) befestigt ist und nahe dem proximalen Ende (28) des Spritzenzylinders zur Injektion von Flüssigkeit oder nahe dem distalen Ende (22) zum Abziehen von Flüssigkeit positioniert ist, charakterisiert dadurch, dass der besagte Kolben aus einer starren Kolbenstangenspitze (70, 130, 150, 160, 180, 204, 210, 220, 230) und einem elastomeren Kolbenring (40, 140, 190, 200, 211, 221, 231) besteht, wobei der besagte elastomere Kolbenring (40, 140, 190, 200, 211, 221, 231) aus einer Innenwand (100) und einer Außenwand (120) besteht,
(1) die besagte Innenwand (100) umfasst mindestens einen Innenring (102), der sich leicht in Richtung des Ringzentrums vorwölbt und so geformt ist, dass er eine Vertiefung oder eine Kerbe (86, 152, 162, 184, 186) in besagtem zylindrischen Schaft (80) der besagten starren Kolbenstangenspitze (70, 130, 150, 160, 180, 204, 210, 220, 230) bildet und damit eine nicht bewegliche Innendichtung formt;
(2) besagte Außenwand (120), die mindestens eine Kolbenringrippe (144, 146, 206, 208, 214, 216, 218, 224, 226, 228, 234, 236, 238, 240) umschließt, die dazu dient, eine zweite bewegliche Dichtung mit der Innenwand (21) des besagten Spritzenzylinders (20) zu bilden.

2. eine Spritze (10) gemäß Anspruch 1, wobei die besagte starre Kolbenstangenspitze (70, 130, 150, 160, 180, 204, 210, 220, 230) wie ein Pilz geformt ist und sich wie folgt zusammensetzt:
(i) aus dem zylindrischen Schaft (80) mit einem distalen Ende (82) und einem proximalen Ende (84) und der Vertiefung oder der Kerbe (86, 152, 162, 184, 186) zwischen dem besagten distalen Ende (82) und dem besagten proximalen Ende (84), die so aufgebaut ist, dass sie den besagten elastomeren Kolbenring (40, 140, 190, 200, 211, 221, 231) aufnimmt und damit die nicht bewegliche Innendichtung bildet.
(ii) einem konisch geformten Kopf (90), der mit besagtem zylindrischen Schaft (80) aus einem Stück besteht, wobei besagter konisch geformter Kopf (90) einen Außendurchmesser aufweist, der nur geringfügig kleiner ist als der Innendurchmesser des besagten Spritzenzylinders (20), was die freie Bewegung des besagten konisch geformten Kopfes (90) in besagtem Spritzenzylinder (20) ermöglicht.
(iii) besagtem elastomeren Kolbenring (40, 140, 190, 200, 211, 221, 231) befestigt an besagtem zylindrischen Schaft (80) der besagten starren Kolbenstangenspitze (70, 130, 150, 160, 180, 204, 210, 220, 230).

3. Eine Spritze gemäß Anspruch 2, wobei der besagte konisch geformte Kopf (ii) (90) einen zirkulären Flansch (92) am proximalen Ende, einen Gipfelpunkt (96) im Mittelpunkt am distalen Ende und den Seiten verbindenden besagten Flansch (92) und besagten Gipfelpunkt (96) aufweist.

4. Eine Spritze (10) gemäß den Ansprüchen 1-3, bei der die Kolbenstangenspitze (70, 130, 150, 160, 180, 204, 210, 220, 230) aus Polymermaterial besteht; besagter Spritzenzylinder (20) besteht aus Glas oder Polymermaterial; der besagte Spritzenzylinder (20) hat:
(I) ein distales Ende, das in einer sich verjüngenden Spitze (24) endet und darin eine Bohrung (26) aufweist, an die eine Injektionsnadel oder ein mit einer Schlauchführung ausgestattetes Verbindungsstück befestigt werden kann; und
(II) einem proximalen Ende (28) zum Einführen des Kolbens.

5. Eine Spritze (10) gemäß den Ansprüchen 1-4, bei der die besagte starre Kolbenstangenspitze (70, 130, 150, 160, 180, 204, 210, 220, 230) weiterhin noch mit einer integralen Kolbenstange (50) am proximalen Ende verbunden ist.

6. Eine Spritze (10) gemäß den Ansprüchen 1-5, bei der die besagte Kolbenstangenspitze noch über weitere Befestigungsmöglichkeiten am proximalen Ende verfügt.

7. Eine Spritze gemäß dem Anspruch 6, wobei es sich bei den besagten Befestigungsmöglichkeiten um Muttergewinde (164) in besagter Kolbenstangenspitze (70, 130, 150, 160, 180, 204, 210, 220, 230) handelt.

8. Eine Spritze (10) gemäß den Ansprüchen 1-7, bei der die besagte Kolbenstangenspitze (70, 130, 150, 160, 180, 204, 210, 220, 230) zwei oder mehrere Vertiefungen oder Kerben (86, 152, 162, 184, 186) umfasst, um so den besagten elastomeren Kolbenring aufzunehmen und damit die nicht beweglichen Dichtungen zu bilden.

9. Eine Spritze (10) gemäß dem Anspruch 8, wobei besagter elastomerer Kolbenring zwei oder mehrere Kolbenringrippen (144, 146, 206, 208, 214, 216, 218, 224, 226, 228, 234, 236, 238, 240) umfasst, um so sekundäre bewegliche Dichtungen mit der Innenwandung des besagten Spritzenzylinders zu bilden.

10. Eine Spritze (10) gemäß den Ansprüchen 1-9, bei der die besagte Kolbenstangenrippe horizontal in Richtung der Innenwandung des besagten Spritzenzylinders ausgerichtet ist.

11. Eine Spritze gemäß den Ansprüchen 1-9, bei der die besagte Kolbenringrippe in Richtung des distalen Endes des Spritzenzylinders (22) ausgerichtet ist.

12. Eine Spritze gemäß den Ansprüchen 1-9, bei der die besagte Kolbenringrippe in Richtung des proximalen Endes des Spritzenzylinders (28) ausgerichtet ist.

13. Eine Spritze gemäß den Ansprüchen 1-9, bei der zumindest eine der besagten Kolbenringrippen in Richtung des distalen Endes (22) des Spritzenzylinders und mindestens eine der besagten Kolbenringrippen in Richtung des proximalen Endes (28) des Spritzenzylinders zeigt.

14. Eine Spritze gemäß den Ansprüchen 1-9, die drei Kolbenringrippen umfasst, wobei eine davon horizontal ausgerichtet, die zweite in Richtung des distalen Endes des Spritzenzylinders und die dritte in Richtung des proximalen Endes ausgerichtet ist.

15. Eine Spritze gemäß den Ansprüchen 1-14, bei der der besagte Spritzenzylinder aus einem Material besteht, das undurchlässig für ein inertes Gas ist und aus der Stoffgruppe ausgewählt wird, die Polyethylen, Polypropylen, Polystyrole, Acrylpolymere und Methacrylpolymere beinhaltet.

16. Eine Spritze gemäß den Ansprüchen 1-15, bei der die besagte Kolbenringspitze aus thermoplastischem Material besteht.

17. Eine Spritze gemäß den Ansprüchen 1-16, bei der die besagte Kolbenstangenspitze aus einem Material besteht, ausgewählt aus der Stoffgruppe, die Polyethylen, Polypropylen, Polystyrole, Acrylpolymere und Methacrylpolymere beinhaltet.

18. Eine Spritze gemäß den Ansprüchen 1-17, bei der der besagte Kolbenring aus komprimierbarem, elastomerem Material besteht, das aus der Stoffgruppe ausgewählt wird, die natürliche und synthetische Gummiverbindungen beinhaltet

19. Eine Spritze gemäß den Ansprüchen 1-18, bei der das besagte Material aus der Stoffgruppe ausgewählt wird, die wie folgt beinhaltet:
natürliches Gummi
Acrylatbutadien-Gummi
Cis-Polybutadien
Chlorobutyl-Gummi
Chlorhaltige Polyethylenelastomere
Polyalkylenoxidpolymere
Ethylenvinylacetat
Fluorosilicon-Gummiverbindungen
Hexafluoropropylen-Vinyliden
Tetrafluoroethylen-Terpolymere
Butyl-Gummiverbindungen
Polyisobuten
Synthetisches Polyisopren-Gummi
Silicongummiverbindungen
Styrolbutadien-Gummiverbindungen
Tetrafluoroethylenpropylen-Copolymere und thermoplastische Copolyester

20. Eine Spritze gemäß den Ansprüchen 1-19, bei der es sich bei der besagten Flüssigkeit um eine medizinische Flüssigkeit handelt.

21. Eine Spritze gemäß dem Anspruch 20, wobei es sich bei der besagten medizinischen Flüssigkeit um ein diagnostisches Kontrastmittel handelt.

22. Eine Kartusche (10), optional vorbefüllt und gedacht zur Injektion einer Flüssigkeit oder zum Abziehen einer Flüssigkeit, bestehend aus:
(a) einem Kartuschenzylinder (20), der eine Innenfläche (21) umfasst, die eine zylindrische Kammer zur Aufnahme einer Flüssigkeit definiert;
(b) einem Kolben, der beweglich in besagtem Zylinder (20) befestigt und nahe dem proximalen Ende (28) des Zylinders positioniert ist zur Injektion einer Flüssigkeit, der dadurch charakterisiert ist, dass der besagte Kolben eine starre Kolbenstangenspitze (70, 130, 150, 160, 180, 204, 210, 220, 230) und einen elastomeren Kolbenring (40, 140, 190, 200, 211, 221, 231) besitzt, wobei der besagte elastomere Kolbenring (40, 140, 190, 200, 211, 221, 231) eine Innenwand (100) und eine Außenwand (120) besitzt;
(1) die besagte Innenwand (100) umfasst mindestens einen Innenring (102), der sich leicht in Richtung des Ringzentrums vorwölbt und dazu dient, eine Vertiefung oder eine Kerbe (86, 152, 162, 184, 186) in einem zylindrischen Schaft (80) der besagten starren Kolbenstangenspitze zu bilden und damit eine nicht bewegliche Innendichtung zu formen;
(2) besagte Außenwand (120), die zumindest eine Kolbenringrippe (144, 146, 206, 208, 214, 216, 218, 224, 226, 228, 236, 238, 240) umfasst, die dazu dient, eine sekundäre bewegliche Dichtung mit der Innenwand (21) des besagten Zylinders (20) zu bilden.

23. Eine Kartusche (10) gemäß dem Anspruch 22, bei der die besagte starre Kolbenstangenspitze (70, 130, 150, 160, 180, 204, 210, 220, 230) eine pilzähnliche Form hat und sich wie folgt zusammensetzt:
(i) dem zylindrischen Schaft (80) der ein distales Ende (82) und ein proximales Ende (84) und die Vertiefung oder Kerbe (86, 152, 162, 184, 186) zwischen dem besagten distalen und besagtem proximalen Ende aufweist, die dazu dient, den besagten elastomeren Kolbenring (40, 140, 190, 200, 211, 221, 231) aufzunehmen und damit die nicht bewegliche Innendichtung zu bilden;
(ii) einem konisch geformten Kopf (90) der mit besagtem zylindrischen Schaft (80) aus einem Stück besteht, wobei der besagte konisch geformte Kopf einen Außendurchmesser hat, der etwas geringer ist als der Innendurchmesser des besagten Zylinders (20) und so eine frei Beweglichkeit des besagten konisch geformten Kopfes (90) in besagtem Zylinder (20) ermöglicht.
(iii) besagtem elastomeren Kolbenring (40, 140, 190, 200, 211, 211, 221, 231), befestigt auf besagtem zylindrischen Schaft (80) der besagten Kolbenstangenspitze.

24. Eine Kartusche (10) gemäß dem Anspruch 23, bei der der besagte konisch geformte Kopf (ii) (90) einen zirkulären Flansch (92) an dessen proximalem Ende besitzt, einen Gipfelpunkt (96) im Zentrum am distalen Ende und einen Seiten verbindenden besagten Flansch (92) und besagten Gipfelpunkt (96).

25. Eine Kartusche (10) gemäß den Ansprüchen 22-24, bei der die besagte Kolbenstangenspitze (70, 130, 150, 160, 180, 204, 210, 220, 230) aus polymerem Material besteht; besagter Zylinder (20) besteht aus Glas oder polymerem Material, wobei sich der besagte Zylinder wie folgt zusammensetzt:
(I) aus einem distalen Ende, das in einer sich verjüngenden Spitze (24) ausläuft und eine Bohrung (26) besitzt, an welcher eine Injektionsnadel oder eine mit einer Schlauchführung ausgestattete Verbindung befestigt werden kann und
(II) einem proximalen Ende (28) zur Aufnahme des Kolbens.

26. Eine Kartusche (10) gemäß den Ansprüchen 22-25, bei der die besagte starre Kolbenstangenspitze noch eine integrale Kolbenstange (50) an ihrem proximalen Ende besitzt.

27. Eine Kartusche gemäß den Ansprüchen 22-26, bei der die besagte Kolbenstangenspitze noch weitere Befestigungsmöglichkeiten am proximalen Ende aufzeigt.

28. Eine Kartusche gemäß dem Anspruch 27, bei der die besagten Befestigungsmöglichkeiten Muttergewinde (164) in besagter Kolbenstangenspitze beinhalten.

29. Eine Kartusche gemäß den Ansprüchen 22-28, bei der die besagte Kolbenstangenspitze zwei oder mehrere Vertiefungen oder Kerben aufweist, um den besagten elastomeren Kolbenring aufzunehmen und damit eine nicht bewegliche Dichtung zu bilden.

30. Eine Kartusche gemäß dem Anspruch 29, bei der der besagte elastomere Kolbenring zwei oder mehrere Kolbenringrippen umfasst, um so sekundäre bewegliche Dichtungen mit der Innenwand des Zylinders zu bilden.

31. Eine Kartusche gemäß den Ansprüchen 22-30, bei der die besagte Kolbenringrippe horizontal in Richtung der Innenwand des besagten Zylinders ausgerichtet ist.

32. Eine Kartusche gemäß den Ansprüchen 22-30, bei der die besagte Kolbenstangenrippe in Richtung des distalen Endes des Zylinders ausgerichtet ist.

33. Eine Kartusche gemäß den Ansprüchen 22-30, bei der zumindest eine der besagten Kolbenringrippen in Richtung distalem Ende des Zylinders und mindestens eine besagte Kolbenringrippe in Richtung proximalem Zylinderende ausgerichtet ist.

34. Eine Kartusche gemäß den Ansprüchen 22-30, die drei Kolbenringrippen umfasst, wobei eine horizontal, die zweite in Richtung distalem Zylinderende und die dritte in Richtung proximalem Ende des Zylinders ausgerichtet ist.

35. Eine Kartusche gemäß den Ansprüchen 22-34, bei der der besagte Kartuschenzylinder aus einem Material besteht, das für inerte Gase undurchlässig ist und aus der Stoffgruppe ausgewählt wird, die Polyethylen, Polypropylen, Polystyrole, Acrylpolymere und Methacrylpolymere beinhaltet.

36. Eine Kartusche gemäß den Ansprüchen 22-35, bei der die besagte Kolbenstangenspitze aus thermoplastischem Material besteht.

37. Eine Kartusche gemäß den Ansprüchen 22-36, bei der die besagte Kolbenstangenspitze aus Material besteht, dass aus der Stoffgruppe ausgewählt wird, die Polyethylen, Polypropylen, Polystyrole, Acrylpolymere und Methacrylpolymere beinhaltet.

38. Eine Kartusche gemäß den Ansprüchen 22-37, bei der der besagte Kolbenring aus komprimierbarem elastomerem Material besteht, das aus der Stoffgruppe ausgewählt wird, die natürliche und synthetische Gummiverbindungen umfasst.

39. Eine Kartusche gemäß den Ansprüchen 22-38, bei der das elastomere Material aus der Gruppe ausgewählt wird, die wie folgt enthält:
natürliches Gummi
Acrylatbutadien-Gummi
Cis-Polybutadien
Chlorobutyl-Gummi
Chlorhaltige Polyethylenelastomere
Polyalkylenoxidpolymere
Ethylenvinylacetat
Fluorosilicon-Gummiverbindungen
Hexafluoropropylen-Vinyliden
Tetrafluoroethylen-Terpolymere
Butyl-Gummiverbindungen
Polyisobuten
Synthetisches Polyisopren-Gummi
Silicongummiverbindungen
Styrolbutadien-Gummiverbindungen
Tetrafluoroethylenpropylen-Copolymere und thermoplastische Copolyester

40. Eine Kartusche gemäß den Ansprüchen 22-39, wobei es sich bei der besagten Flüssigkeit um eine medizinische Injektionsflüssigkeit handelt.

41. Eine Kartusche gemäß dem Anspruch 40, wobei es sich bei der besagten medizinischen Injektionsflüssigkeit um ein diagnostisches Kontrastmittel handelt.

## Revendications

1. Seringue (10), éventuellement préremplie, destinée à injecter un fluide dans un site ou à extraire un fluide d'un site, comprenant :
(a) un tube de seringue (20) ayant une surface intérieure (21) qui définit une chambre cylindrique pour contenir un fluide ;
(b) un plongeur monté de façon coulissante dans ledit tube (20) et placé près de l'extrémité proximale (28) du tube pour injecter un fluide dans un site, ou placé près de l'extrémité distale (22) du tube pour extraire un fluide d'un site ;
**caractérisée en ce que** ledit plongeur comprend un embout de tige de plongeur rigide (70, 130, 150, 160, 180, 204, 210, 220, 230) et un anneau de plongeur en élastomère (40,140, 190, 200, 211, 221, 231), dans laquelle ledit anneau de plongeur en élastomère (40, 140, 190, 200, 211, 221, 231) comporte une paroi intérieure (100) et une paroi extérieure (120),
(1) ladite paroi intérieure (100) comprend au moins un anneau intérieur (102) légèrement en saillie vers le centre de l'anneau, prévu pour s'engager dans un évidement ou une gorge (86, 152, 162, 184, 186) d'une queue cylindrique (80) dudit embout de tige de plongeur rigide (70, 130, 150, 160, 180, 204, 210, 220, 230) et pour former un joint interne non glissant avec cette dernière ;
(2) ladite paroi extérieure (120) comprend au moins une nervure annulaire de plongeur (144, 146, 206, 208, 214, 216, 218, 224, 226, 228, 234, 236, 238, 240) prévue pour former un joint coulissant secondaire avec la paroi intérieure (21) dudit tube (20).

2. Seringue (10) selon la revendication 1, dans laquelle ledit embout de tige de plongeur rigide (70, 130, 150, 160, 180, 204, 210, 220, 230) a une configuration en champignon, comprenant :
(i) la queue cylindrique (80) ayant une extrémité distale (82) et une extrémité proximale (84) et l'évidement ou gorge (86, 152, 162, 184, 186), entre ladite extrémité distale (82) et ladite extrémité proximale (84), prévu pour recevoir ledit anneau de plongeur en élastomère (40, 140, 190, 200, 211, 221, 231) et formant un joint interne non glissant avec ce dernier ;
(ii) une tête conique (90) solidaire de ladite queue cylindrique (80), ladite tête conique (90) ayant un diamètre extérieur légèrement plus petit que le diamètre intérieur dudit tube (20) pour permettre un mouvement libre de ladite tête conique (90) dans ledit tube (20) ;
(iii) ledit anneau de plongeur en élastomère (40, 140, 190, 200, 211, 221, 231) monté sur ladite queue cylindrique (80) dudit embout de tige de plongeur rigide (70, 130, 150, 160, 180, 204, 210, 220, 230).

3. Seringue (10) selon la revendication 2, dans laquelle ladite tête conique (ii) (90) comporte une collerette circulaire (92) sur son extrémité proximale, un sommet (96) au centre de son extrémité distale, et une face reliant ladite collerette (92) et ledit sommet (96).

4. Seringue (10) selon les revendications 1 à 3, dans laquelle ledit embout de tige de plongeur rigide (70, 130, 150, 160, 180, 204, 210, 220, 230) est fabriqué en matière polymère, et ledit tube de seringue (20) est fabriqué en verre ou en matière polymère, ledit tube (20) ayant :
(I) une extrémité distale se terminant par un bout conique (24) comportant un trou (26) dans ce dernier, auquel une aiguille d'injection ou un connecteur équipé d'un conduit tubulaire peut être attaché ; et
(II) une extré mité proximale (28) pour recevoir un plongeur.

5. Seringue (10) selon les revendications 1 à 4, dans laquelle ledit embout de tige de plongeur rigide (70, 130, 150, 160, 180, 204, 210, 220, 230) comprend en outre une tige de plongeur solidaire (50) à son extrémité proximale.

6. Seringue (10) selon les revendications 1 à 5, dans laquelle ledit embout de tige de plongeur comprend en outre un moyen de fixation à son extré mité proximale.

7. Seringue selon la revendication 6, dans laquelle ledit moyen de fixation comprend un filetage femelle (164) dans ledit embout de tige de plongeur (70, 130, 150, 160, 180, 204, 210, 220, 230).

8. Seringue (10) selon les revendications 1 à 7, dans laquelle ledit embout de tige de plongeur (70, 130, 150, 160, 180, 204, 210, 220, 230) comprend deux évidements ou gorges (86, 152, 162, 184, 186), ou davantage, pour recevoir ledit anneau de plongeur en élastomère afin de former des joints d'étanchéité non glissants.

9. Seringue (10) selon la revendication 8, dans laquelle ledit anneau de plongeur en élastomère comprend deux nervures d'anneau de plongeur (144, 146, 206, 208, 214, 216, 218, 224, 226, 228, 234, 236, 238, 240), pour former des joints glissants secondaires avec la paroi intérieure dudit tube.

10. Seringue (10) selon les revendications 1 à 9, dans laquelle ladite nervure d'anneau de plongeur est orientée horizontalement vers la paroi intérieure dudit tube.

11. Seringue selon les revendications 1 à 9, dans laquelle ladite nervure d'anneau de plongeur est orientée vers l'extrémité distale du tube (22).

12. Seringue selon les revendications 1 à 9, dans laquelle ladite nervure d'anneau de plongeur est orientée vers l'extrémité proximale du tube (28).

13. Seringue selon les revendications 1 à 9, dans laquelle au moins une desdites nervures d'anneau de plongeur est orientée vers l'extrémité distale (22) du tube et au moins une desdites nervures d'anneau de plongeur est orientée vers l'extrémité proximale (28) du tube.

14. Seringue selon les revendications 1 à 9, comprenant trois nervures annulaires de plongeur dont l'une est orientée horizontalement, la deuxième est orientée vers l'extrémité distale du tube et la troisième est orienté e vers l'extrémité proximale du tube.

15. Seringue selon les revendications 1 à 14, dans laquelle ledit tube de seringue est fabriqué en une matière imperméable aux gaz inertes, choisie dans le groupe comprenant le polyéthylè ne, le polypropylè ne, les polystyrènes, les polymères acryliques et les polymères méthacryliques.

16. Seringue selon les revendications 1 à 15, dans laquelle ledit embout de tige de plongeur est fabriqué en une matière thermoplastique.

17. Seringue selon les revendications 1 à 16, dans laquelle ledit embout de tige de plongeur est fabriqué en une matière choisie dans le groupe comprenant le polyéthylène, le polypropylène, les polystyrènes, les polymères acryliques et les polymères méthacryliques.

18. Seringue selon les revendications 1 à 17, dans laquelle ledit anneau de plongeur est fabriqué en une matière élastomère compressible choisie dans le groupe comprenant les caoutchoucs naturels et synthétiques.

19. Seringue selon les revendications 1 à 18, dans laquelle ladite matière élastomère est choisie dans le groupe comprenant :
caoutchouc naturel ;
caoutchouc acrylate-butadiène
cis-polybutadiène ;
caoutchouc chlorobutyle ;
élastomères de type polyéthylène chloré ;
polymères d'oxyde de polyalkylène ;
éthylène-acétate de vinyle ;
caoutchoucs de fluorosilicone ;
hexafluoropropylène-vinylidène ;
terpolymères de tétrafluoroéthylène ;
caoutchouc butyle ;
polyisobutène ;
caoutchouc polyisoprène synthétique ;
caoutchoucs de silicone ;
caoutchoucs styrène-butadiène ;
copolymères de tétrafluoroé thylène-propylène ; et
copolyesters thermoplastique.

20. Seringue selon les revendications 1 à 19, dans laquelle ledit fluide est un fluide médical.

21. Seringue selon la revendication 20, dans laquelle ledit fluide médical est un milieu de contraste pour diagnostic.

22. Cartouche (10) éventuellement préremplie, prévue pour injecter un fluide dans un site ou extraire un fluide d'un site, comprenant :
(a) un tube de cartouche (20) présentant une surface intérieure (21) qui définit une chambre cylindrique pour contenir un fluide ;
(b) un plongeur monté de façon coulissante dans ledit tube (20) et placé près de l'extrémité proximale (28) du tube pour injecter un fluide dans un site ;
**caractérisée en ce que** ledit plongeur comprend un embout de tige de plongeur rigide (70, 130, 150, 160, 180, 204, 210, 220, 230) et un anneau de plongeur en élastomère (40,140, 190, 200, 211, 221, 231), dans laquelle ledit anneau de plongeur en élastomère (40, 140, 190, 200, 211, 221, 231) comporte une paroi intérieure (100) et une paroi extérieure (120),
(1) ladite paroi intérieure (100) comprend au moins un anneau intérieur (102) légèrement en saillie vers le centre de l'anneau, prévu pour s'engager dans un évidement ou une gorge (86, 152, 162, 184, 186) d'une queue cylindrique (80) dudit embout de tige de plongeur rigide (70, 130, 150, 160, 180, 204, 210, 220, 230) et pour former un joint interne non glissant avec cette dernière ;
(2) ladite paroi extérieure (120) comprend au moins une nervure annulaire de plongeur (144, 146, 206, 208, 214, 216, 218, 224, 226, 228, 234, 236, 238, 240) prévue pour former un joint coulissant secondaire avec la paroi intérieure (21) dudit tube (20).

23. Cartouche (10) selon la revendication 22, dans laquelle ledit embout de tige de plongeur rigide (70, 130, 150, 160, 180, 204, 210, 220, 230) a une configuration en champignon comprenant :
(i) la queue cylindrique (80) ayant une extrémité distale (82) et une extrémité proximale (84) et l'évidement ou gorge (86, 152, 162, 184, 186), entre ladite extrémité distale (82) et ladite extré mité proximale (84), prévu pour recevoir ledit anneau de plongeur en élastomère (40, 140, 190, 200, 211, 221, 231) et formant un joint interne non glissant avec ce dernier ;
(ii) une tête conique (90) solidaire de ladite queue cylindrique (80), ladite tête conique (90) ayant un diamètre extérieur légèrement plus petit que le diamètre intérieur dudit tube (20) pour permettre un mouvement libre de ladite tête conique (90) dans ledit tube (20) ;
(iii) ledit anneau de plongeur en élastomère (40, 140, 190, 200, 211, 221, 231) monté sur ladite queue cylindrique (80) dudit embout de tige de plongeur rigide.

24. Cartouche (10) selon la revendication 23, dans laquelle ladite tête conique (ii) (90) comporte une collerette circulaire (92) sur son extrémité proximale, un sommet (96) au centre de son extrémité distale, et une face reliant ladite collerette (92) et ledit sommet (96).

25. Cartouche (10) selon les revendications 22 à 24, dans laquelle ledit embout de tige de plongeur rigide (70, 130, 150, 160, 180, 204, 210, 220, 230) est fabriqué en matière polymère, et ledit tube de seringue (20) est fabriqué en verre ou en matière polymère, ledit tube (20) ayant :
(I) une extrémité distale se terminant par un bout conique (24) et ayant un trou (26) dans ce dernier, auquel une aiguille d'injection ou un connecteur équipé d'un conduit tubulaire peut être attaché ; et
(II) une extré mité proximale (28) pour recevoir un plongeur.

26. Cartouche (10) selon les revendications 22 à 25, dans laquelle ledit embout de tige de plongeur rigide comprend en outre une tige de plongeur rigide (50) à son extré mité proximale.

27. Cartouche selon les revendications 22 à 26, dans laquelle ledit embout de tige de plongeur comprend en outre un moyen de fixation à son extrémité proximale.

28. Cartouche selon la revendication 27, dans lequel ledit moyen de fixation comprend un filetage femelle (164) dans ledit embout de tige de plongeur.

29. Cartouche selon les revendications 22 à 28, dans laquelle ledit embout de tige de plongeur comprend deux évidements ou rainures, ou davantage, pour recevoir ledit anneau de plongeur en élastomère de manière à former des joints non glissants avec celui-ci.

30. Cartouche selon la revendication 29, dans laquelle ledit anneau de plongeur en élastomère comprend deux nervures d'anneau de plongeur ou davantage, pour former des joints glissants secondaires avec la paroi intérieure dudit tube.

31. Cartouche selon les revendications 22 à 30, dans laquelle ladite nervure d'anneau de plongeur est orientée horizontalement vers la paroi intérieure dudit tube.

32. Cartouche selon les revendications 22 à 30, dans laquelle ladite nervure d'anneau de plongeur est orientée vers l'extré mité distale du tube.

33. Cartouche selon les revendications 22 à 30, dans laquelle au moins une desdites nervures d'anneau de plongeur est orientée vers l'extrémité distale du tube et au moins une desdites nervures d'anneau de plongeur est orientée vers l'extrémité proximale du tube.

34. Cartouche selon les revendications 22 à 30, comprenant trois nervures annulaires de plongeur dont l'une est orientée horizontalement, la deuxième est orienté vers l'extrémité distale du tube et la troisième est orientée vers l'extrémité proximale du tube.

35. Seringue selon les revendications 22 à 24, dans laquelle ledit tube de seringue est fabriqué en une matière imperméable aux gaz inertes, choisie dans le groupe comprenant le polyéthylène, le polypropylène, les polystyrènes, les polymères acryliques et les polymères méthacryliques.

36. Seringue selon les revendications 22 à 35, dans laquelle ledit embout de tige de plongeur est fabriqué en une matière thermoplastique.

37. Seringue selon les revendications 22 à 36, dans laquelle ledit embout de tige de plongeur est fabriqué en une matière choisie dans le groupe comprenant le polyéthylène, le polypropylène, les polystyrènes, les polymères acryliques et les polymères méthacryliques.

38. Seringue selon les revendications 22 à 37, dans laquelle ledit anneau de plongeur est fabriqué en une matière élastomère compressible choisie dans le groupe comprenant les caoutchoucs naturels et synthétiques.

39. Seringue selon les revendications 22 à 38, dans laquelle ladite matière élastomère est choisie dans le groupe comprenant :
caoutchouc naturel ;
caoutchouc acrylate-butadiène
cis-polybutadiène ;
caoutchouc chlorobutyle ;
élastomères de type polyéthylène chloré ;
polymères d'oxyde de polyalkylène ;
éthylène-acétate de vinyle ;
caoutchoucs de fluorosilicone ;
hexafluoropropylène-vinylidène ;
terpolymères de tétrafluoroéthylène ;
caoutchouc butyle ;
polyisobutène ;
caoutchouc polyisoprène synthétique ;
caoutchoucs de silicone ;
caoutchoucs styrène-butadiène ;
copolymè res de tétrafluoroéthylène-propylène ; et
copolyesters thermoplastiques.

40. Cartouche selon les revendications 22 à 39, dans laquelle ledit fluide est un fluide médical injectable.

41. Cartouche selon la revendication 40, dans laquelle ledit fluide médical est un milieu de contraste pour diagnostic.
